# EUROPEAN PATENT SPECIFICATION

(11) **EP 0 871 656 B1**
(45) Date of publication and mention of the grant of the patent: **25.09.2002**
(21) Application number: 96918661.8
(22) Date of filing: 03.06.1996
(51) Int. Cl.: C07K 7/06, A61K 38/08

(54) **DOLASTATIN DERIVATIVES, THEIR PREPARATION AND USE**
DERIVATE VON DOLESTATIN, DEREN HERSTELLUNG UND VERWENDUNG
DERIVES DE LA DOLASTATINE, LEURS PREPARATION ET LEURS UTILISATION

(30) Priority: 07.06.1995 US 472453
(43) Date of publication of application: 21.10.1998
(73) Proprietor: Abbott GmbH & Co. KG, 65205 Wiesbaden (DE)
(72) Inventor: HAUPT, Andreas, Westborough, MA 01581 (US); EMLING, Franz, D-67065 Ludwigshafen (DE); ROMERDAHL, Cynthia, A., Wayland, MA 01778 (US)
(74) Representative: Riedl, Peter, Dr.
(86) International application number: PCT/EP96/02393
(87) International publication number: WO 96/040752

(56) References cited:
- WO-A-93/23424
- TETRAHEDRON, vol. 49, no. 41, 8 October 1993, OXFORD GB, pages 9151-9170, XP002015205 G R PETTITT ET AL.: "Isolation of dolastatins 10-15 from the mnarine mollusc Dolabella auricularia "

## Description

The present invention relates to dolastatin derivatives, their preparation and use as well as to pharmaceutical compositions containing said derivatives.

It is known that peptides isolated from marine origin like Dolastatin-10 (US 4,816,444) and Dolastattn-15 (EP-A-398558) show potent cell growth inhibitory activity (cf.: Biochem. Pharmacology 40, no. 8, 1859-64, 1990); J. Natl. Cancer Inst. 85, 483-88, 1993 and references cited therein). Based upon interesting results in experimental tumor systems in vivo, further preclinical evaluation of these natural products is currently under way in order to initiate clinical studies in cancer patients.
However, the natural products have disadvantages, such as poor solubility in aqueous solvents and costly building blocks needed for synthesis.

WO 93/23424 describes derivatives of dolastatin having the formula 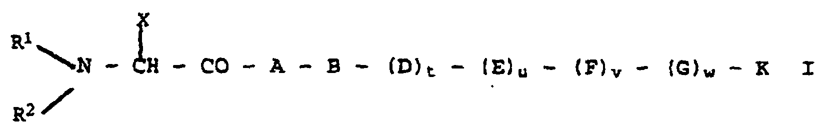 where
- R¹: is alkoxy; alkyl; cycloalkyl; alkylsulfonyl; fluoroalkyl; trifluoroacetyl; amidino; ureyl; piperidinosulfonyl; morpholinosulfonyl; benzyloxycarbonyl; alkyloxycarbonyl; aminosulfonyl which may be substituted by alhyl; hydroxy; arylsulfonyl which may be substituted by one or more substituents independently selected from alkyl, -N(CH₃)₂, nitro, halogen and CF₃; benzyl which may be substituted by up to three substituents independently selected from alkyl, alkoxy, nitro, halogen and CF₃; or NR³R⁴ where R³ and R⁴ may each be either hydrogen or alkyl;
- R²: is hydrogen; alkyl; fluoroalkyl; cycloalkyl; acyl; benzoyl or benzyl which may both be substituted by up to three substituents independently selected from nitro, halogen, CF₃, alkyl and alkoxy
- R¹-N-R²: together may be phthalimido, a 5- or 6-membered heterocycle which may be unsubstituted or substituted with one or more substituents independently selected from phenyl, benzyl, alkyl, N(CH₃)₂, nitro, thienyl, CONH₂ and COOEt
- A: is a valyl, isoleucyl, leucyl, allo-isoleucyl, α-aminoisobutanoyl, 3-tert-butylalanyl, 2-tert-butylglycyl, 3-cyclohexylalanyl, 2,4-diaminobutanoyl, omithyl, lysyl, 2-ethylglycyl, 2-cyclohexylglycyl, norleucyl, norvalyl or arginyl residue;
- B: is a N-alkyl-valyl, -norvalyl, -leucyl; -isoleucyl, -2-tert-butylglycyl, -3-tert-butylalanyl, -3-cyclohexylalanyl, -phenylalanyl, or -2-cyclohexylglycyl residue;
- D,E,F and G: are independently selected from the group consisting of prolyl, homo-prolyl, hydroxyprolyl, thiazolidinyl-4-carbonyl, 1-aminopentyl-1-carbonyl, valyl, 2-tert-butylglycyl, isoleucyl, leucyl, 3-cyclohexylalanyl, phenylalanyl, N-methylphenylalanyl, tetrahydroisoquinolyl-2-carbonyl, 3-thiazolylalanyl, 3-thienylalanyl, histidyl, 1-aminoindyl-l-carbonyl, 2,4-diaminobutanoyl, arginyl, 3-pyridylalanyl, 3-tert-butylalanyl, 2-cyclohexylglycyl, lysyl, norvalyl, norleucyl and 3-naphthylalanyl residues
- X: is hydrogen, alkyl, cycloalkyl, -CH₂-Cyclohexyl or arylalkyl
A and B together, F and G together, R¹R²N-CHX-CO and A together, E and F together, either alone or in pairs, may be 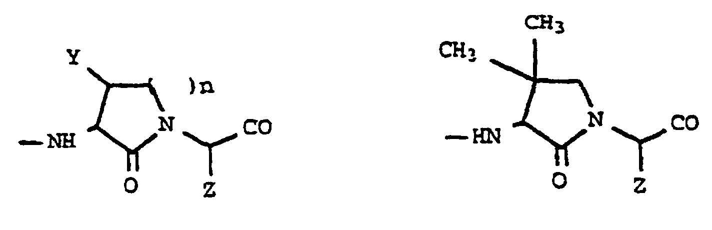 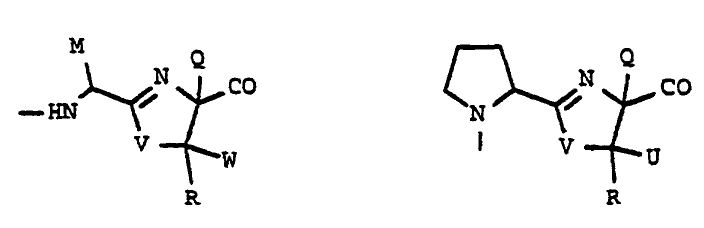 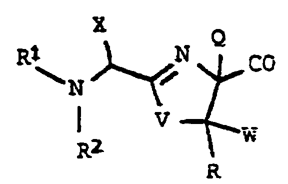 where
- Y: is hydrogen or lower alkyl; Z is hydrogen or lower alkyl; n is 1, 2, or 3; V is oxygen or sulfur; M is hydrogen, lower alkyl, arylalkyl, cyclohexyl, or -CH₂-cyclohexyl; Q is hydrogen; R is hydrogen or lower alkyl; or R and Q may together form a bond; U is hydrogen, lower alkyl, phenyl, or cycloalkyl; and W is hydrogen, lower alkyl or phenyl;
- t,u,v,and w: are independently 0 or 1; and
- K: is hydroxy, alkoxy, phenoxy, benzyloxy or a substituted or unsubstituted amino moiety;
provided that where t, u, v and w are 0, K is not a hydroxy, alkoxy, benzoxy or phenoxy moiety; and further provided that where t, u and v are 0, K is not a hydroxy or alkoxy moiety;
and the salts thereof with physiologically tolerated acids.

The invention described herein provides novel peptides and derivatives thereof which offer improved therapeutic potential for the treatment of neoplastic diseases as compared to Dolastatin-15. Furthermore, the compounds of this invention may be conveniently synthesized as described in detail below. formula I

R¹R²N-CHX-CO-A-B-D-E-(F)ₜ-K I

where
- R¹: is methyl, ethyl, or isopropyl;
- R²: is hydrogen, methyl, or ethyl;
- R¹-N-R²: together may be a pyrrollidine ring;
- A: is a valyly, isoleucyl, leucyl, 2-tert-butylglycyl, 2-ethylglycyl, norleucyl or norvalyl residue;
- B: is a N-methyl-valyl, -leucyl, -isoleucyl, -norvalyl, -norleucyl -2-tert-butylglycyl, -3-tert-butylalanyl, or -2-ethylglycyl residue;
- D: is a prolyl, 3,4-dehydroprolyl, 4-fluoroprolyl, 4,4-difluoroprolyl, azetidine-2-carbonyl, homoprolyl, 3-methylprolyl, 4-methylprolyl, 5-methylprolyl, or thiazolidine-4-carbonyl residue;
- E: is a 3,4-dehydroprolyl, 4-fluoroprolyl, 3-methylprolyl, 4-methylprolyl, azetidine-2-carbonyl, or 4,4-difluoroprolyl residue;
- F: is a valyl, 2-tert-butylglycyl, isoleucyl, leucyl, 2-cyclohexylglycyl, norleucyl, norvalyl, neopentylglycyl, alanyl, β-alanyl, or aminoisobutyroyl residue;
- X: is alkyl (preferably C₂₋₅), cyclopropyl, or cyclopentyl;
- t: is 0 or 1; and
- K: is alkoxy (preferably C₁₋₄), benzyloxy or a substituted or unsubstituted amino moiety
of the formula R⁵-N-R⁶ wherein
- R⁵: is hydrogen, or hydroxy, or C₁₋₄ alkoxy, or benzyloxy (which may be substituted by up to two substituents which may independently be CF₃, nitro, C₁₋₄ -alkyl-sulfonyl, C₁₋₄-alkoxy, halogen, C₁₋₄-alkyl, cyano, hydroxy, N(CH₃)₂, COOMe, COOEt, COOiPr, or COONH₂), or phenyloxy (which may be substituted by up to two substituents which may independently be CF₃, nitro, C₁₋₄-alkylsulfonyl, C₁₋₄-alkoxy, halogen, C₁₋₄-alkyl, cyano, hydroxy, N(CH₃)₂, COOMe, COOEt, COOiPr, or CONH₂);
- R⁶: is hydrogen, or C₁₋₁₂-alkyl (which may be substituted by one or more fluoro atoms), or -C(CH₃)₂CN, or -C(CH₃)₂CCH, or -C(CH₃)₂C=CH₂, or -C(CH₃)₂CH₂OH, or -C(CH₃)₂CH₂CH₂OH, or -(CH₂)ᵥ-C₃₋₇-cycloalkyl (v=0,1,or 2) (which may be substituted by a methyl group), or norephedryl, or norpseudo-ephedryl, or quinolyl, or pyrazyl, or adamantyl, or -CH₂-benzimidazolyl, or -CH₂-adamantyl, or alphamethyl-benzyl, or alpha-dimethylbenzyl, or -(CH₂)ᵥ-phenyl (v=0,1,2,or 3; which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or -(CH₂)ₘ-naphthyl (m=0 or 1; which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or -(CH₂)_{w}-benzhydryl (w=0,1, or 2; which may be substituted by up to two substituents which may independently be CF₃, nitro, C₁₋₄-alkylsulfonyl, C₁₋₄-alkoxy, halogen, C₁₋₄-alkyl which may. form a cyclic system, cyano, hydroxy, N(CH₃)₂, COOMe, COOEt, COOiPr, or CONH₂), or
biphenyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or pyridyl (which may be substituted by up to two substitutents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
picolyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
-CH₂-CH₂-pyridyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or benzothiazolyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or benzoisothiazolyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or benzopyrazolyl (which may be substituted by up to two substituents which may independently be CF₃, nitro, C₁₋₇-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or benzoxazolyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or -(CH₂)ₘ-fluorenyl (m=0 or 1; which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or pyrimidyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or -(CH₂)ₘ-indanyl (m=0 or 1; which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or -(CH₂CH₂O)_{y}-CH₃ (y=0,1,2,3,4, or 5), or - (CH₂CH₂O)_{y}-CH₂CH₃ (y=0,1,2,3,4, or 5), or -NH-C₆H₅ (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
-NCH₃-C₆H₅ (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
-NH-CH₂-C₆H₅ (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
-NCH₃-CH₂-C₆H₅ (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
5-membered heteroaryl which may be substituted by up to two substituents which may independently be CF₃, nitro, C₁₋₄-alkylsulfonyl, C₁₋₄-alkoxy, thiometbyl, thioethyl, picolyl, acetyl, C₃₋₆-cycloalkyl, thiophenyl, -CH₂-COOEt, C₃₋₄-alkylen group forming a bicyclic system with the heterocycle, phenyl (which may be substituted by up to two substituents which may independently be nitro, CF₃, CN, halogen, or
C₁₋₄-alkyl), benzyl (which may be substituted by up to two substituents which may independently be nitro, CF₃, halogen, C₁₋₄-alkyl, C₁₋₄-alkylsulfonyl, cyano, hydroxy, C₁₋₄-dialkylamino), or
-CHR⁷-5-membered heteroaryl (which may be substituted by up to two substituents which may indenpendently be CF₃, nitro, cyano, halogen, COOMe, COOEt, COOiPr, CONH₂, C₁₋₄-alkyl, C₁₋₄-alkoxy, phenyl, benzyl, naphthyl, or C₁₋₄-alkylsulfonyl [R⁷ = hydrogen, linear or branched C₁₋₅ alkyl, benzyl]).
-CHR⁷-5-membered heteroaryl may for example be represented by one of the folloging residues: 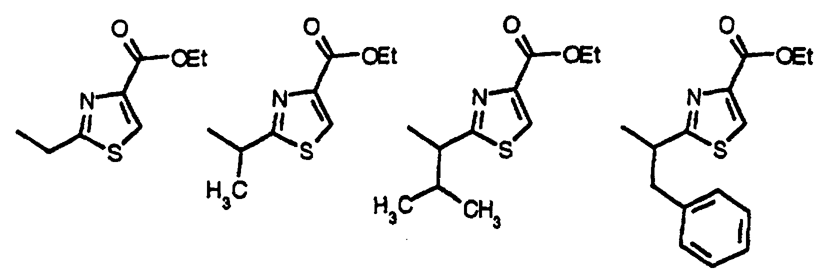 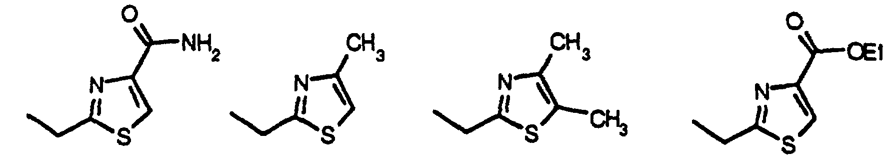 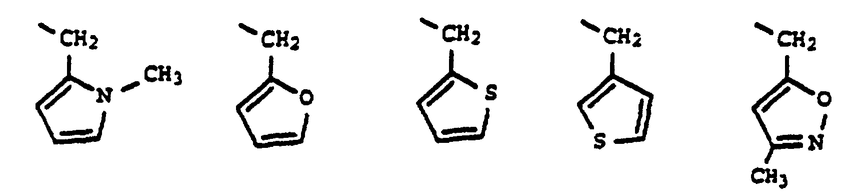 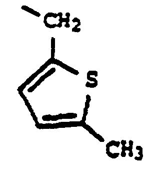 5-membered heteroaryl may for example be represented by the following residues: 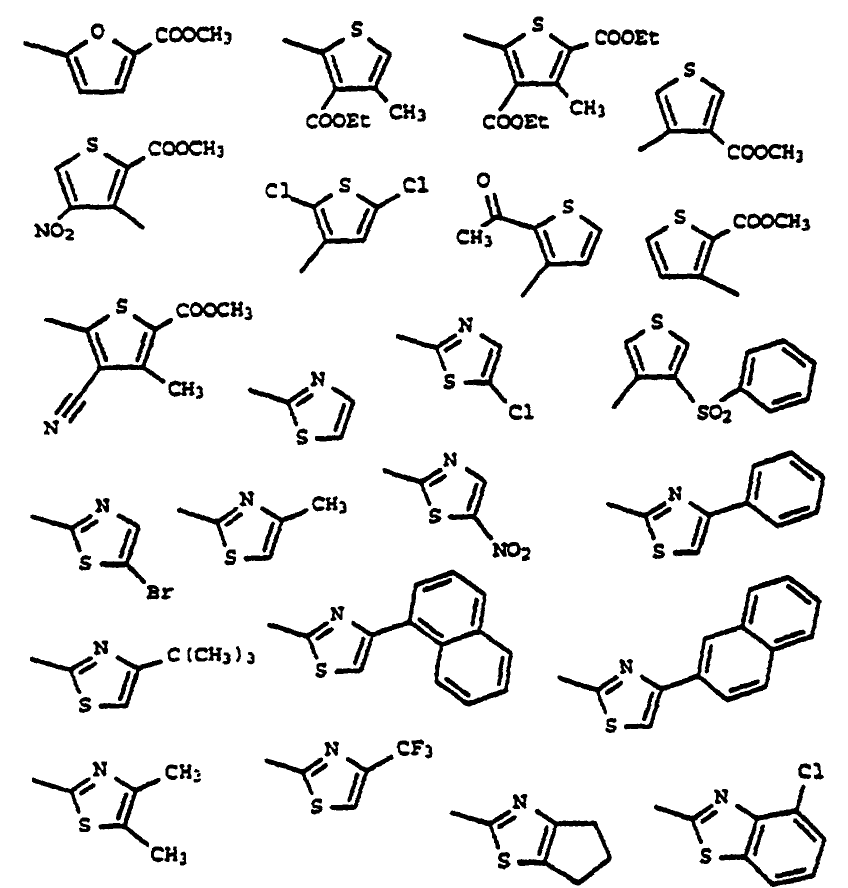 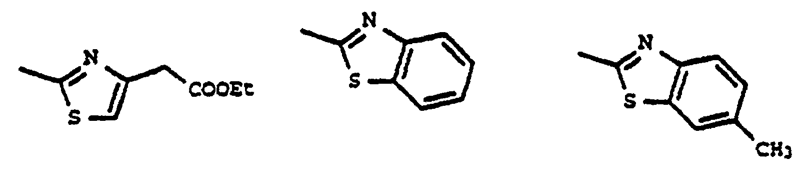 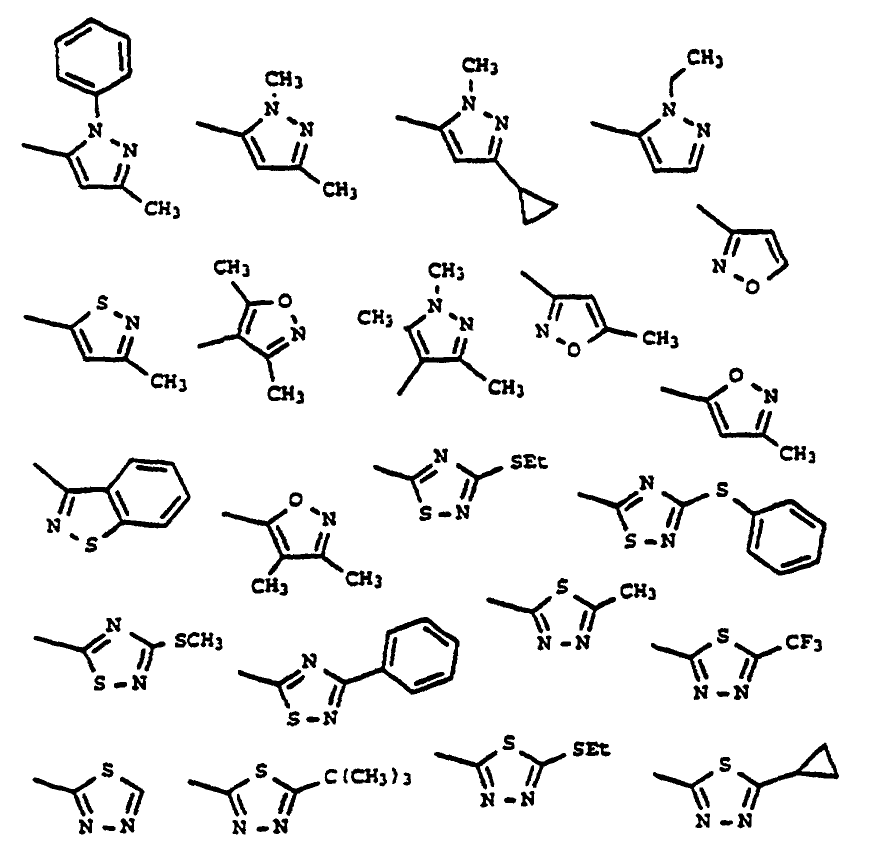 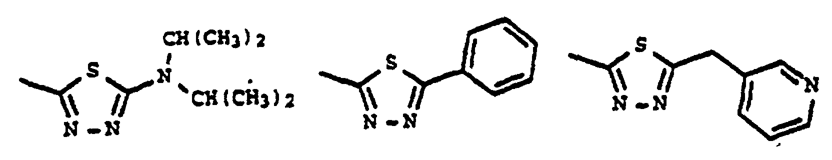 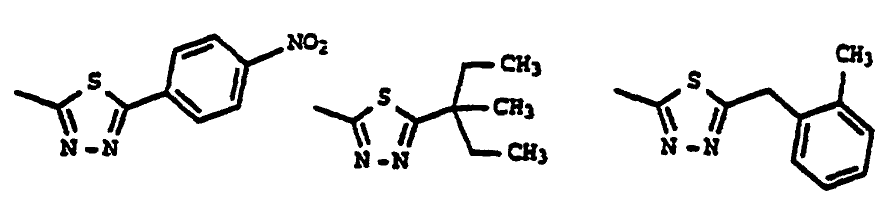 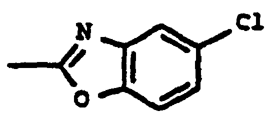 R⁵-H-R⁶ together may also form structures selected from the group consisting of

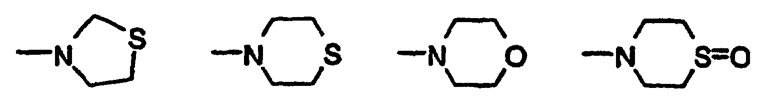

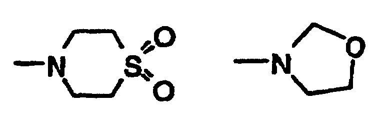

Compounds of this invention also include the salts of the peptides with physiologically tolerated acids.

This invention also provides methods for preparing the compounds of formula I, pharmaceutical compositions containing such compounds together with a pharmaceutically acceptable carrier and methods for using same for treating cancer in mammals.

Preferred are compounds of the formula I where the substituents R¹, R², A, B, D, E, X, F and t have the following meanings
- R¹: is methyl or ethyl;
- R²: is hydrogen, methyl, or ethyl;
- A: is a valyl, isoleucyl, 2-tertbutylglycyl residue;
- B: is a N-methyl-valyl, -isoleucyl, or -2-tert-butylglycyl residue;
- D: is a prolyl, 3,4-dehydroprolyl, 4-fluoroprolyl, 3-methylprolyl or thiazolidine-4-carbonyl residue;
- E: is a 3,4-dehydroprolyl, 4-fluoroprolyl, 3-methylprolyl, or azetidine-2-carbonyl residue;
- F: is a D-valyl, 2-tert-butylglycyl, D-isoleucyl, D-leucyl, or aminoisobutyroyl residue;
- X: is -CH(CH₃)₂, -C(CH₃)₃, or -CH(CH₃)CH₂CH₃;
- t: is 0 or 1;

Preferred meanings for K are:
-OC(CH₃)₃, -NH₂, -NH-C₁₋₁₂-alkyl, -NH-C₃₋₈-cycloalkyl, -NH-[3,3,0]-bicyclooctyl, norephedryl, norpseudoephedryl, -NH-quinolyl, -NH-pyrazyl, -NH-CH₂-benzimidazolyl, -NH-adamantyl, -NH-CH₂-adamantyl, -NH-CH(CH₃)-phenyl, -NH-C(CH₃)₂-phenyl, -N(C₁₋₄-alkoxy)C₁₋₄-alkyl, -N(C₁₋₄-alkoxy)-CH₂-phenyl, -N(C₁₋₄-alkoxy)-phenyl, -N(CH₃)OBzl, -NH-(CH₂)ᵥ-phenyl (v=0,1,2,or 3), -NH-(CH₂)ₘ-naphthyl (m=0 or 1), -NH-(CH₂)_{w}-benzhydryl (w=0,1, or 2), -NH-biphenyl, -NH-pyridyl, -NH-CH₂-pyridyl, -NH-CH₂-CH₂-pyridyl, -NH-benzothiazolyl, -NH-benzoisothiazolyl, -NH-benzopyrazolyl, -NH-benzoxazolyl, -NH-(CH₂)ₘ-fluorenyl (m=0 or 1), -NH-pyrimidyl, -NH-(CH₂)ₘ-indanyl (m=0 or 1), -NH-(CH₂CH₂O)_{y}-CH₃ (y=0,1,2,3,4, or 5), -NH-(CH₂CH₂O)_{y}-CH₂CH₃ (y=0,1,2,3,4, or 5), -NH-(5-membered heteroaryl) (as shown on pages 7-9), -NH-CHR⁷-5-membered heteroaryl [R⁷ = hydrogen, linear or branched C₁₋₅ alkyl, benzyl] (as shown on page 6); or K is

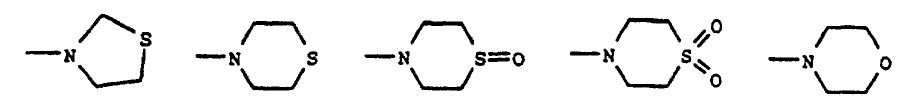

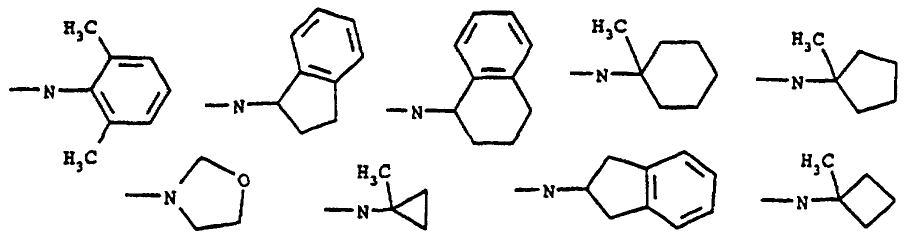

More preferred K is:
-NHCH₃, -NHCH₂CH₃, -NH(CH₂)₂CH₃, -NH(CH₂)₃CH₃, -NH(CH₂)₄CH₃, -NH(CH₂)₅CH₃, -NH(CH₂)₆CH₃, -NH(CH₂)₇CH₃, -NHCH(CH₃)_{2,} -NHCH(CH₃)CH₂CH₃, -NHCH(CH₃)CH₂CH₂CH₃, -NHCH(CH₂CH₃)₂, -NH(CH₂CH₂CH₃)₂, -NHC(CH₃)₃, -NHCH(CH₂CH₃)CH₂CH₂CH₃, -NHCH(CH₃)CH(CH₃)₂, -NHCH(CH₂CH₃)CH(CH₃)₂, -NHCH(CH₃)C(CH₃)₃, -NH-cyclopropyl, -NH-cyclobutyl, -NH-cyclopentyl, -NH-cyclohexyl, -NH-cycloheptyl, -NH-cyclooctyl, -NH-bicyclo[3,3,0]octyl, -N(CH₃)OCH₃, -N(CH₃)OCH₂CH₃, -N(CH₃)OCH₂CH₂CH₃, -N(CH₃)OCH(CH₃)₂, -N(CH₂CH₃)OCH_{3,} -N(CH₂CH₃)OCH₂CH₃ -N(CH(CH₃)₂)OCH₃, -N(CH₃)OCH₂C₆H₅, -N(OCH₃)CH₂-C₆H₅, -N(CH₃)OC₆H₅, -NH-CH₂-C₆H₅, -NH(CH₂)₂C₆H_{5,} -NH(CH₂)₃C₆H₅, -NHCH(CH₃)C₆H₅, -NHC(CH₃)₂C₆H₅, -NHC(CH₃)₂CH₂CH₃, -NHC(CH₃)(CH₂CH₃)₂, -NHCH(CH₃)CH(OH)C₆H₅, -NHCH₂-cyclohexyl, -NH-CH₂CF₃, -NHCH(CH₂F)₂, -NHC(CH₃)₂CH₂CH₂OH, -NH(CH₂CH₂O)₂CH₂CH₃, -NHC(CH₃)₂CH(CH₃)₂, -NHC(CH₃)₂CN, -NHC(CH₃)₂CCH, -NHC(CH₃)₂C=CH₂, norephedryl, norpseudoephedryl, -NH-quinolyl, -NH-pyrazyl, -NH-adamantyl(1), -NH-adamantyl(2), -NH-CH₂-adamantyl, -NH-CH₂-naphthyl, -NH-benzhydryl, -NH-biphenyl, -NH-pyridyl, -NH-picolyl, -NH-CH₂-CH₂-pyridyl, -NH-benzothiazolyl, -NH-benzoisothiazolyl, -NH-benzopyrazolyl, -NH-benzoxazolyl, -NE-fluorenyl, -NH-pyrimidyl, -NH-thiazolyl(2), -NH-isoxazolyl(3), -NH-CH₂-furanyl(2), -NH-(3-methyl)isoxazolyl(5), -NH-(3-methyl)isothiazolyl(5), -NH-(2-trifluormethyl)-thiadiazolyl(5), -OC(CH₃)₃, -NH-CH₂-(4-methyl)thiazolyl(2), -NH-CH₂-thienyl(2), -NH-CH₂-(5-methyl)thienyl(2), -NH-(2-methyl)-thiadiazolyl(5),-NH-(2-cyclopropyl)thiadiazolyl(5), or K is

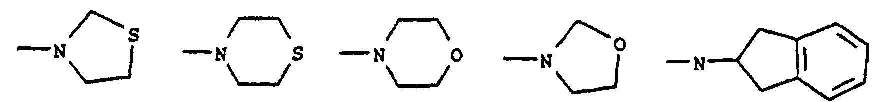

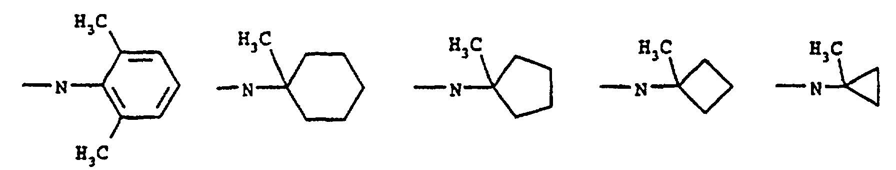

These examples illustrate but do not limit the scope of the present invention.

The peptides of the formula I are composed of L-amino acids but F may also be a D-amino acid.

The new compounds may be present as salts with physiologically tolerated acids such as: hydrochloric acid, citric acid, tartaric acid, lactic acid, phosphoric acid, methanesulfonic acid, acetic acid, formic acid, maleic acid, fumaric acid, malic acid, succinic acid, malonic acid, sulfuric acid, L-glutamic acid, L-aspartic acid, pyruvic acid, mucic acid, benzoic acid, glucuronic acid, oxalic acid, ascorbic acid and acetylglycine.

The novel compounds can be prepared by known methods of peptide chemistry. Thus, the peptides can be assembled sequentially from amino acids or by linking suitable small peptide fragments. In the sequential assemblage, starting at the C-terminus the peptide chain is extended stepwise by one amino acid each time. In fragment coupling it is possible to link together fragments of different lengths, and the fragments in turn can be obtained by sequential assemblage from amino acids or themselves by fragment coupling.

Both in the sequential assemblage and in the fragment coupling it is necessary to link the units by forming an amide linkage. Enzymatic and chemical methods are suitable for this.

Chemical methods for forming the amide linkage are described in detail by Mueller, Methoden der organischen Chemie Vol. XV/2, pp 1 to 364, Thieme Verlag, Stuttgart, 1974; Stewart, Young, Solid Phase Peptide Synthesis, pp 31 to 34, 71 to 82, Pierce Chemical Company, Rockford, 1984; Bodanszky, Klausner, Ondetti, Peptide Synthesis, pp 85 to 128, John Wiley & Sons, New York, 1976 and other standard works on peptide chemistry. Particular preference is given to the azide method, the symmetric and mixed anhydride method, in situ generated or preformed active esters, the use of urethane protected N-carboxy anhydrides of amino acids and the formation of the amide linkage using coupling reagents/activators, especially dicyclohexylcarbodiimide (DCC), diisopropylcarbodiimide (DIC ), 1-ethoxycarbonyl-2-ethoxy-1,2-dihydroquinoline (EEDQ), 1-ethyl-3-(3-dimethylaminopropyl)carbodiimide hydrochloride (EDCI), n-propanephosphonic anhydride (PPA), N,N-bis(2-oxo-3-oxazolldinyl)-amidophosphoryl chloride (BOP-Cl), bromo-tris-pyrrolidinophosphonium hexafluorophosphate (PyBrop), diphenylphosphoryl azide (DPPA), Castro's reagent (BOP, PyBop), O-benzotriazolyl-N,N,N',N'-tetramethyluronium salts (HBTU), O-Azabenzotriazolyl-N,N,N',N'-tetramethyluronium salts (HATU), diethylphosphoryl cyanide (DEPCN), 2,5-diphenyl-2,3-dihydro-3-oxo-4-hydroxythiophene dioxide (Steglich's reagent; HOTDO) and 1,1'-carbonyldiimidazole (CDI). The coupling reagents can be employed alone or in combination with additives such as N,N-dimethyl-4-aminopyridine (DMAP), N-hydroxy-benzotriazole (HOBt), N-hydroxybenzotriazine (HOOBt), Azabenzotriazole (HOAt), N-hydroxysuccinimide (HOSu) or 2-hydroxypyridine.
Whereas it is normally possible to dispense with protective groups in enzymatic peptide synthesis, reversible protection of reactive groups not involved in formation of the amide linkage is necessary for both reactants in chemical synthesis. Three conventional protective group techniques are preferred for the chemical peptide synthesis: the benzyloxycarbonyl (Z), the t-butoxycarbonyl (Boc) and the 9-fluorenylmethoxycarbonyl (Fmoc) techniques. Identified in each case is the protective group on the alpha-amino group of the chain-extending unit. A detailed review of amino-acid protective groups is given by Mueller, Methoden der organischen Chemie Vol. XV/1, pp 20 to 906, Thieme Verlag, Stuttgart, 1974. The units employed for assembling the peptide chain can be reacted in solution, in suspension or by a method similar to that described by Merrifield in J. Amer. Chem. Soc. 85 (1963) 2149.
Particularly preferred methods are those in which peptides are assembled sequentially or by fragment coupling using the Z, Boc or Fmoc protective group technique.
One of the reactants in the said Merrifield technique is being bonded to an insoluble polymeric support (also called resin hereinafter). This typically entails the peptide being assembled sequentially on the polymeric support using the Boc or Fmoc protective group technique, the growing peptide chain being covalently bonded at the C terminus to the insoluble resin particles (cf. Fig. 1 and 2). This procedure makes it possible to remove reagents and byproducts by filtration, and thus recrystallization of intermediates is unnecessary.

The protected amino acids can be linked to any suitable polymers, which merely have to be insoluble in the solvents used and to have a stable physical form which makes filtration easy. The polymer must contain a functional group to which the first protected amino acid can be firmly attached by a covalent bond. Suitable for this purpose are a wide variety of polymers, eg. cellulose, polyvinyl alcohol, polymethacrylate, sulfonated polystyrene, chloromethylated styrene/divinylbenzene copolymer (Merrifield resin), 4-methylbenzhydrylamine resin (MBHA-resin), phenylacetamidomethyl-resin (Pam-resin), p-benzyloxy-benzyl-alcohol-resin, benzhydryl-amine-resin (BHA-resin), 4-(hydroxymethyl)-benzoyloxy-methyl-resin, the resin of Breipohl et al. (Tetrahedron Letters 28 (1987) 565; supplied by BACHEM), 4-(2,4-dimethoxyphenylaminomethyl)phenoxy-resin (supplied by Novabiochem) or o-chlorotrityl-resin (supplied by Biohellas).

Suitable for peptide synthesis in solution are all solvents which are inert under the reaction conditions, especially water, N,N-dimethylformamide (DMF), dimethyl sulfoxide (DMSO), acetonitrile, dichloromethane (DCM), 1,4-dioxane, tetrahydrofuran (THF), N-methyl-2-pyrrolidone (NMP) and mixtures of the said solvents. Peptide synthesis on the polymeric support can be carried out in all inert organic solvents in which the amino-acid derivatives used are soluble. However, preferred solvents additionally have resin-swelling properties, such as DMF, DCM, NMP, acetonitrile and DMSO, and mixtures of these solvents. After synthesis is complete, the peptide is cleaved off the polymeric support. The conditions under which cleavage off the various resin types is possible are disclosed in the literature. The cleavage reactions most commonly used are acid- and palladium-catalyzed, especially cleavage in liquid anhydrous hydrogen fluoride, in anhydrous trifluoromethanesulfonic acid, in dilute or concentrated trifluoroacetic acid, palladium-catalyzed cleavage in THF or THF-DCM mixtures in the presence of a weak base such as morpholine or cleavage in acetic acid-dichloromethanetrifluoroethanol mixtures. Depending on the chosen protective groups, these may be retained or likewise cleaved off under the cleavage conditions.
Partial deprotection of the peptide may also be worthwhile when certain derivatization reactions are to be carried out. Peptides dialkylated at the N-terminus can be prepared a) by coupling of the appropriate N,N-di-alkylamino acids in solution or on the polymeric support, b) by reductive alkylation of the resin-bound peptide in DMF/1% acetic acid with NaCNBH₃ and the appropriate aldehyde or ketone, c) by hydrogenation of the peptides in solution in the presence of aldehyde or ketone and Pd/C.

The various non-naturally occurring amino acids disclosed herein may be obtained from commercial sources or synthesized from commercially-available materials using methods known in the art. Azetidine-2-carboxylic acid, 3-methyl-L-proline, 5-methyl-L-proline, and Boc- or Fmoc-protected 3,4-dehydroproline are commercially available starting materials (ACROS, NOVABIOCHEM, BACHEM). Cis- and trans-4-fluoroproline can be prepared via a method described by Panasik et al. (N. Panasik, E.S. Eberhardt, A.S. Edison, D.R. Powell, R.T. Raines, Int. J. Peptide Protein Res. 44, 1994, 262-269) from hydroxyproline.

The compounds of this invention may be used to inhibit or otherwise treat solid tumors (e.g. tumors of the lung, breast, colon, prostate, bladder, rectum, or endometrial tumors) or hematological malignancies (e.g. leukemias, lymphomas) by administration of the compound to the mammal.

It is a special advantage of the new compounds that they are more resistant to enzymatic degredation as compared to Dolastatin-15.

Administration may be by any of the means which are conventional for pharmaceutical, preferably oncological, agents, including oral and parenteral means such as subcutaneously, intravenously, intramuscularly and intraperitoneally.

The compounds may be administered alone or in the form of pharmaceutical compositions containing a compound of formula I together with a pharmaceutically accepted carrier appropriate for the desired route of administration. Such pharmaceutical compositions may be combination products, i.e., may also contain other therapeutically active ingredients.

The dosage to be administered to the mammal will contain an effective tumor-inhibiting amount of active ingredient which will depend upon conventional factors including the biological activity of the particular compound employed; the means of administration; the age, health and body weight of the recipient; the nature and extent of the symptoms; the frequency of treatment; the administration of other therapies; and the effect desired. A typical daily dose will be about 0.5 to 50 milligrams per kilogram of body weight on oral administration and about 0.05 to 20 on parenteral administration.

The novel compounds can be administered in conventional solid or liquid pharmaceutical administration forms, e.g. uncoated or (film-)coated tablets, capsules, powders, granules, suppositories or solutions. These are produced in a conventional manner. The active substances can for this purpose be processed with conventional pharmaceutical aids such as tablet binders, fillers, preservatives, tablet disintegrants, flow regulators, plasticizers, wetting agents, dispersants, emulsifiers, solvents, sustained release compositions, antioxidants and/or propellant gases (cf. H. Sucker et al.: Pharmazeutische Technologie, Thieme-Verlag, Stuttgart, 1978). The administration forms obtained in this way normally contain 1-90% by weight of the active substance.

The following examples are intended to illustrate the invention. The proteinogenous amino acids are abbreviated in the examples using the known three-letter code. Other abbreviations used: Me₂Val=N,N-dimethylvaline, MeVal=N-methylvaline.

### A. General procedures

I. The peptides claimed in claim 1 are either synthesized by classical solution synthesis using standard Z- and Boc-methodology as described above or by standard methods of solid-phase synthesis using Boc and Fmoc protective group techniques.

### a) Synthetic cycle for the Fmoc protective group technique

| | | |
|---|---|---|
| 1. | DMF washing | 1 x 1 min |
| 2. | 20% piperidine in DMF | 1 x 4 min |
| 3. | 20% piperidine in DMF | 1 x 16 min |
| 4. | DMF washing | 5 x 1 min |
| 5. | Addition of the preactivated protected amino acid (activation by 1 equivalent of TBTU and | |
| | 5 equivalents of DIPEA in DMF); Peptide coupling | 1 x 61 min |
| 6. | DMF washing | 3 x 1 min |
| 7. | if conversion is incomplete, repetition of coupling (back to 5.) | |
| 8. | 10% acetic anhydride in DMF | 1 x 8 min |
| 9. | DMF washing | 3 x 1 min |
| 10. | back to 2. | |

BOP-Cl and PyBrop were used as reagents for coupling of the amino acid following the N-methylamino acids. The reaction times were correspondingly increased including double couplings. In solution synthesis, the use of either Boc-protected amino acid NCA's (N-carboxy anhydrides), Z-protected amino acid NCA's (N-carboxy anhydrides), or the use of pivaloylchloride as condensing agent respectively is most advantageous for this type of coupling.

### II. Reductive alkylation of the N terminus

The peptide-resin prepared as in AIa was deprotected at the N terminus (steps 2-4 in AIa) and then reacted with a 3-fold molar excess of aldehyde or ketone in DMF/1% acetic acid with addition of 3 equivalents of NaCNBH₃. After reaction was complete (negative Kaiser test) the resin was washed several times with water, isopropanol, DMF and dichloromethane.
Reductive alkylation in solution can e.g. be achieved by reaction of the N-terminally deprotected peptides, peptide fragments, or amino acids with the corresponding aldehydes or ketones using NaCNBH₃ or hydrogen-Pd/C.

### III. Work-up of the peptide-resins obtained as in Ib and II

The peptide-resin was dried under reduced pressure and then subjected to treatment with a TFA/water mixture (95:5) for 1,5 hours (Wade, Tregear, Howard Florey Fmoc Workshop Manual, Melbourne 1985). The resin was then filtered off and washed with TFA and DCM. The filtrate and the washings were concentrated, and the peptide was precipitated by addition of diethyl ether. After cooling in an ice bath, the precipitate was filtered off, taken up in 30% acetic acid and lyophilized.

IV. When an o-chlorotrityl-resin (supplied by Biohellas) is used, the suspension of the peptide-resin in an acetic acid/trifluoroethanol/dichloromethane mixture (1:1:3) is stirred at room temperature for 1 h. The resin is then filtered off with suction and thoroughly washed with the cleavage solution. The combined filtrates are concentrated in vacuo and treated with ether. The precipitated solid is removed by filtration or centrifugation, washed with diethyl ether and dried under reduced pressure.

### V. Purification and characterization of the peptides

Purification was carried out by gel chromatography (SEPHADEX G-10, G-15/10% HOAc, SEPHADEX LH20/MeOH) and/or medium pressure chromatography (stationary phase: HD-SIL C-8, 20-45 mikron, 100 Angstrom; mobile phase: gradient with A = 0.1% TFA/water, B = 0.1% TFA/MeOH), or preparative HPLC (stationary phase: Waters Delta-Pak C-18, 15 mikron, 100 Angstrom; mobile phase: gradient with A = 0.1 % TFA/water, B = 0.1 % TFA/MeOH).

The purity of the resulting products was determined by analytical HPLC (stationary phase: 100 2.1 mm VYDAC C-18, 300 Angstrom; mobile phase: acetonitrile-water gradient, buffered with 0.1% TFA, 40°C).
Characterization was by fast atom bombardment mass spectroscopy and NMR spectroscopy.

### B. Specific procedures

### EXAMPLE 1 (SEQ ID NO: 1)

### Me₂Val-Val-MeVal-Pro-L-Azetidinyl-2-carboxamide

0.53 g of Fmoc-RINK-resin (substitution 0.46 mmol/g), corresponding to a batch size of 0.25 mmol, were reacted as in AIa with 0.4 mmol each of
Fmoc-L-Azetidine-2-carboxylic acid
Fmoc-Pro-OH
Pmoc-MeVal-OH
Fmoc-Val-OH
Fmoc-Val-OH

The amino acid following the N-methylamino acid was coupled on with PyBrop as coupling reagent with a double coupling. After the iterative synthetic cycles were completed, the peptide-resin underwent N-terminal deprotection (steps 2-4 in AIa), and was further reacted with aqueous formaldehyde solution as in All and then dried under reduced pressure. The resulting resin was subjected to TFA cleavage as in AIII. The crude product was purified by prep. medium pressure chromatography to yield 5 mg of the desired pure peptide (10-40% A in 10'; 40-90% A in 200'). The compound was further characterized by fast atom bombardment mass spectrometry ([M+H]⁺ = 537.37).

### EXAMPLE 2 (SEQ ID NO: 1)

### Me₂Val-Val-MeVal-Pro-3,4-dehydroprolylbenzylamide

### a) Z-MeVal-Pro-OMe

66.25 g (250 mmol) Z-MeVal-OH were dissolved in 250 ml dry dichloromethane. After addition of 36.41 ml (262.5 mmol) triethylamine , the reaction mixture was cooled to -25° C and 32.27 ml (262.5 mmol) pivaloyl chloride were added. After stirring for 2,5 h, 41.89 g (250 mmol) H-Pro-OMe x HCl in 250 ml dichloromethane, neutralized with 36.41 ml (262.5 mmol) triethylamine at 0°C, were added to the reaction mixture. Stirring continued for 2 h at -25° C and overnight at room temperature. The reaction mixture was diluted with dichloromethane and thoroughly washed with saturated aqueous NaHCO₃ solution (3x), water (1x), 5 % citric acid (3x) and saturated NaCl solution. The organic phase was dried over sodium sulfate and evaporated to dryness. The residue (91.24 g) was stirred with petroleum ether overnight and filtered. 62.3 g of product were obtained.

### b) H-MeVal-Pro-OMe

48.9 g (130 mmol) Z-MeVal-Pro-OMe were dissolved in 490 ml methanol. After addition of 10.9 ml (130 mmol) concentrated hydrochloric acid and 2.43 g 10 % Palladium/charcoal, the reaction mixture was hydrogenated. Filtration and evaporation to dryness yielded 36.43 g of the product.

### c) Z-Val-MeVal-Pro-OMe

18.1 g (65 mmol) H-MeVal-Pro-OMe, 21.6 g (78 mmol) Z-Val-N-carboxyanhydride and 22.8 ml (130 mmol) diisopropylethylamine were stirred in 110 ml DMF at 40° C for 2 d. After evaporation of DMF, dichloromethane was added and the organic phase washed with saturated aqueous NaHCO₃ solution (3x), water (1x), 5 % citric acid (3x) and saturated NaCl solution. The organic phase was dried over sodium sulfate and evaporated to dryness. The product (29.3 g) was obtained as a viscous oil.

### d) H-Val-MeVal-Pro-OMe

29.3 g (61.6 mmol) of Z-Val-MeVal-Pro-OMe were dissolved in 230 ml methanol. After addition of 1.15 g 10 % Palladium/charcoal, the reaction mixture was hydrogenated. Filtration and evaporation to dryness yielded 21.96 g of the product.

### e) Z-Val-Val-MeVal-Pro-OMe

15.29 g (61 mmol) Z-Val-OH and 21.96 g (61 mmol) H-Val-MeVal-Pro-OMe were dissolved in 610 ml dichloromethane and cooled to 0° C. After addition of 8.16 ml (73.2 mmol) N-Methylmorpholine, 2.77 g (20.3 mmol) HOBt and 11.74 g (61 mmol) EDCI, the reaction mixture was stirred overnight at room temperature, diluted with dichloromethane and thoroughly washed with saturated aqueous NaHCO₃ solution (3x), water (1x), 5 % citric acid (3x) and saturated NaCl solution. The organic phase was dried over sodium sulfate and evaporated to dryness to yield 31.96 g of the product.

### f) Z-Val-Val-MeVal-Pro-OH

31.96 g (57 mmol) Z-Val-Val-MeVal-Pro-Ome were dissolved in 250 ml methanol and 102.6 ml (102.6 mmol) 1 M LiOH in water were added. After stirring at room temperature for 24 h, water was added and the methanol evaporated under reduced pressure. The aqueous phase was extracted 3 times with ethyl acetate, adjusted to pH 2 at 4°C, and extracted 3 times with ethyl acetate. The combined organic extracts were dried over sodium sulfate and the solvent evaporated under reduced pressure to yield 30.6 g of a white solid.

### g) Me₂Val-Val-MeVal-Pro-OH

The N,N-dimethylated tetrapeptide acid was prepared from the Z-protected tetrapeptide acid by hydrogenation of the Z protecting group and subsequent addition of aqueous formaldehyde solution to the hydrogenation mixture to yield the desired product in an almost quantitative yield.

### h) Me₂Val-Val-MeVal-Pro-3,4-dehydroproline methylester

3.38 g Me₂Val-Val-MeVal-Pro-OH (7.27 mmol) and 0.925 g 3,4-dehydroproline methylester hydrochloride (7.27 mmol) were dissolved in 75 ml dry dichloromethane. 0.975 ml N-methylmorpholine (8.72 mmol), 0.332 g HOBt (2.43 mmol) and 1.4 g EDCI (7.27 mmol) were added at 4°C. After stirring overnight at room temperature, dichloromethane was added and the organic phase washed 3 times with sat. sodium hydrogen carbonate solution and once with water. The organic layer was extracted with 5 % aqu. citric acid. The pH of the acidic aqueous layer was then adjusted to pH 8 with 1 N NaOH and extracted 4 times with dichloromethane. Drying over sodium sulfate and evaporating under reduced pressure yielded 2.82 g of the pentapeptide methylester.

### k) Me₂Val-Val-MeVal-Pro-3,4-dehydroproline benzylamide

A solution of 2.0 g of the dehydroproline containing pentapeptide methylester in 20 ml methanol was treated with 4.6 ml of a 1 N LiOH solution at room temperature. After the reaction was complete (tlc control), water was added, the methanol evaporated and the compound lyophilized from water. The white powder obtained was dissolved in 36 ml dichloromethane, and 0.388 ml (3.55 mmol) benzylamine were added. After addition of 0.476 ml N-methylmorpholine (4.26 mmol), 0.163 g HOBt (1.19 mmol) and 0.684 g EDCI (3.55 mmol) at 4°C, the reaction mixture was stirred at room temperature overnight. Dichloromethane was added and the organic phase washed 3 times with sat. sodium hydrogen carbonate solution and once with water. The organic layer was extracted 2 times with 5 % citric acid. The acidic aqueous layer was adjusted to pH 9 with 5 N NaOH and extracted 4 times with dichloromethane. Drying over sodium sulfate and evaporating the solvent under reduced pressure yielded 600 mg of crude pentapeptide benzylamide. 300 mg of this crude peptide were further purified using medium pressure liquid chromatography (0-10% A in 5', 10-25% A in 10', 25-90% A in 450') to yield 92 mg of the analytically pure product. The compound was further characterized by fast atom bombardment mass spectrometry ([M+H]⁺ = 639.4).

The following compounds were prepared and can be prepared according to examples 1 and 2:

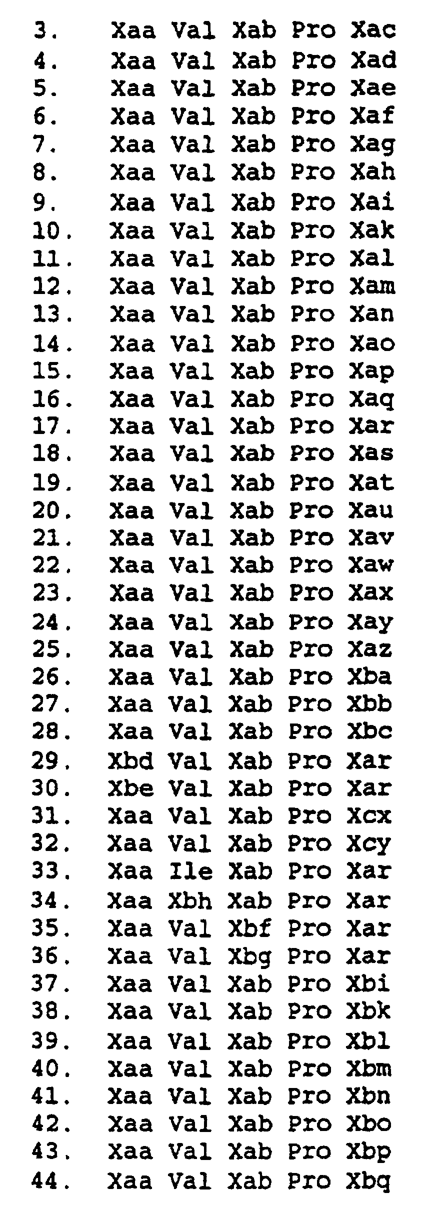

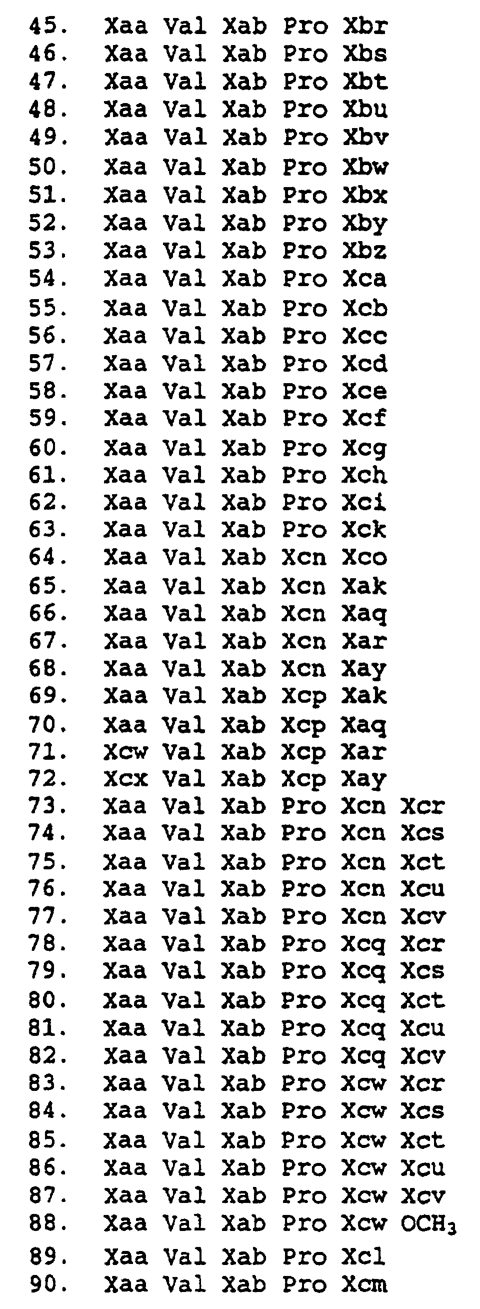

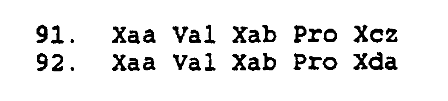

Examples for the MS-characterization of the synthesized novel compounds are given in the following table.

| EXAMPLE [No.] | Fast atom bombardment MS analysis. [Mol.-Weight (measured)] |
|---|---|
| 61. | 627 |
| 64. | 546 |
| 88. | 552 |
| 89. | 655 |

**Table I -**

| Sequence Identification of Compounds Prepared According to Examples 1 and 2 | |
|---|---|
| Compound Number (s) | Sequence ID Number |
| 1-32, 35-63, 88-92 | 1 |
| 33 | 2 |
| 34 | 3 |
| 64-72 | 4 |
| 73-87 | 5 |

The symbols Xaa in the summary have the following meanings:
- Xaa:: N,N-Dimethylvaline
- Xab:: N-Methylvaline

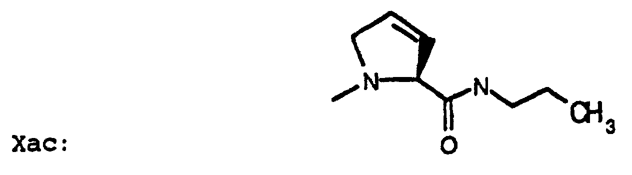

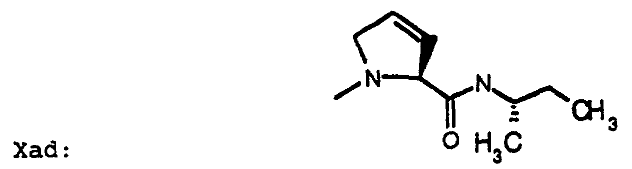

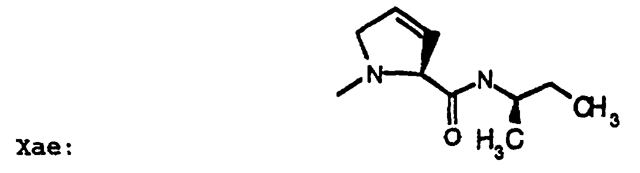

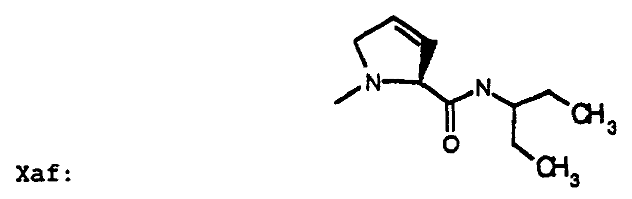

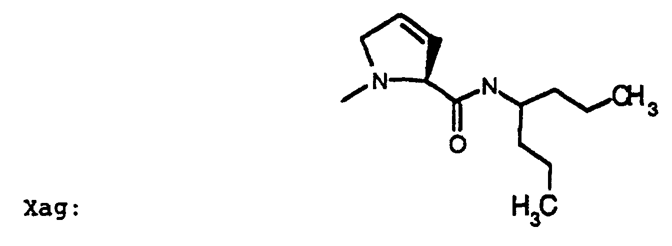

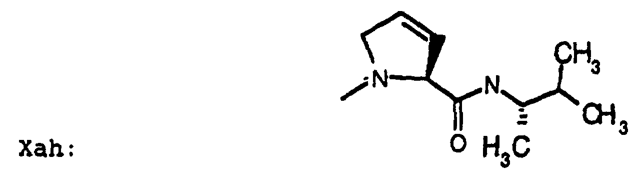

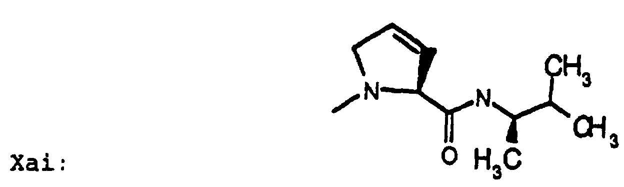

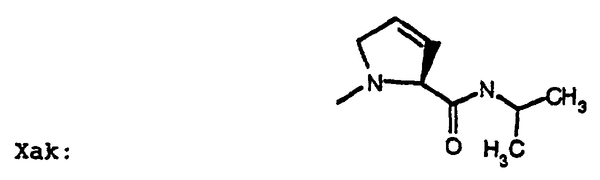

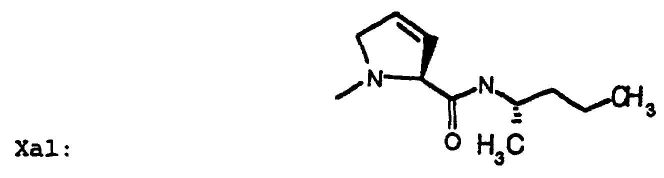

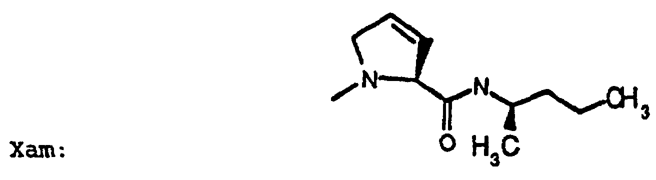

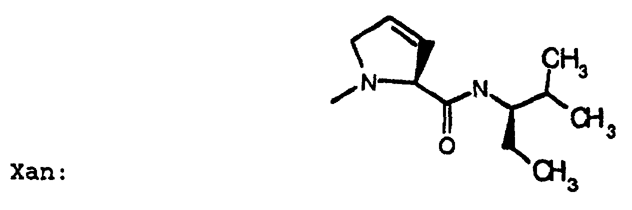

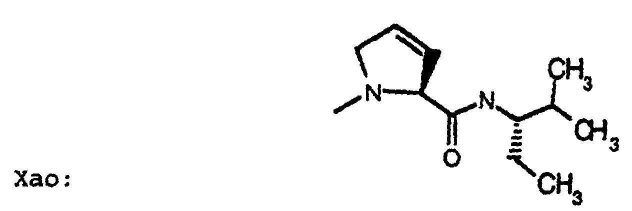

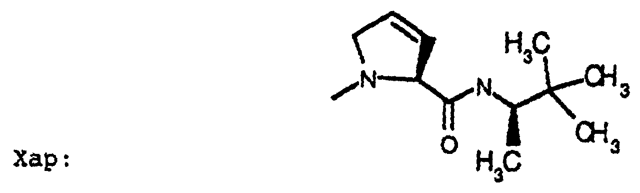

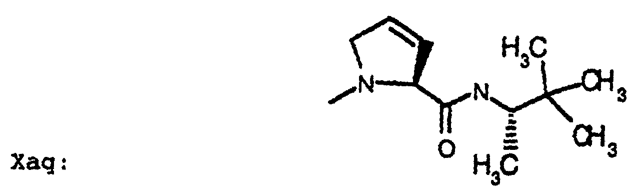

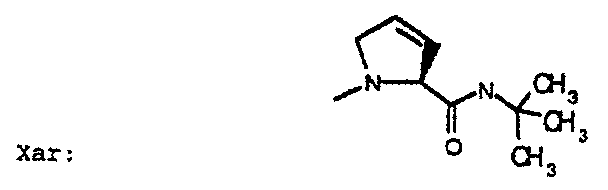

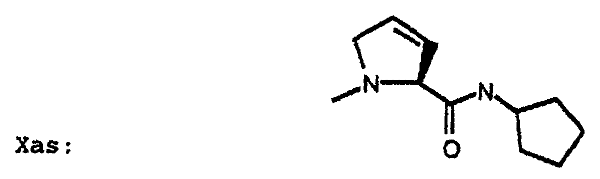

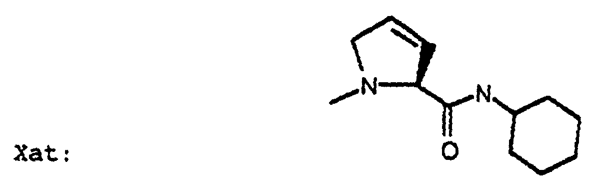

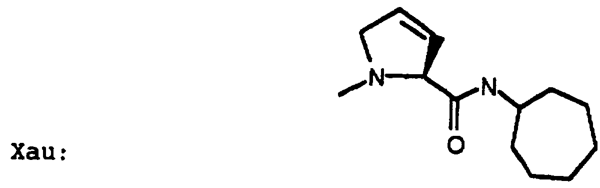

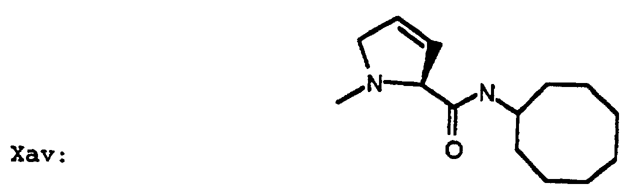

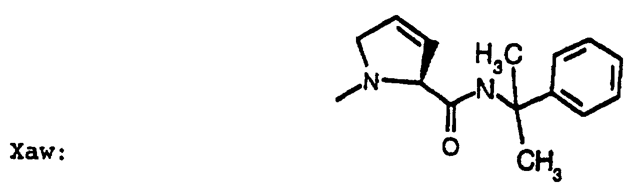

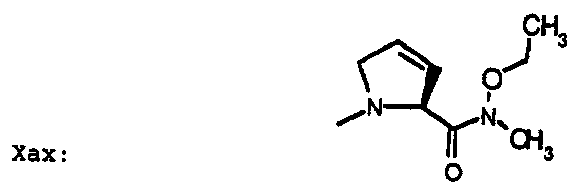

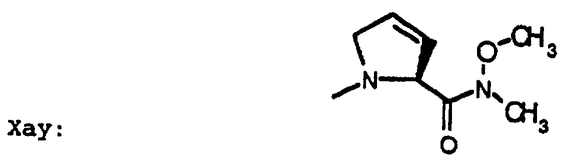

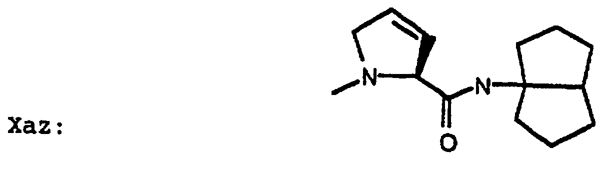

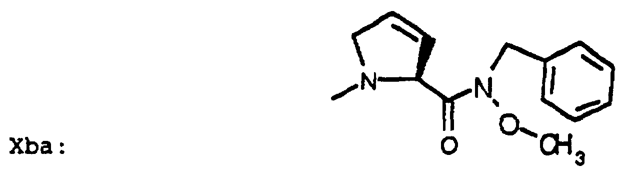

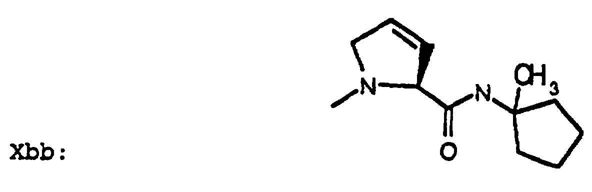

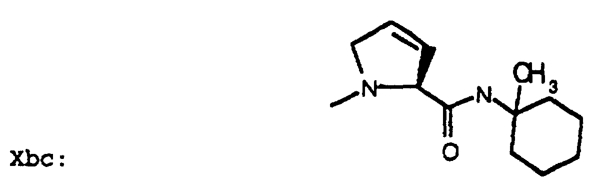

- Xbd:: N,N-dimethylisoleucine
- Xbe:: N,N-dimethyl-2-tert.butyl-glycine
- Xbf:: N-methylisoleucine
- Xbg:: N-methyl-2-tert.butyl-glycine
- Xbh:: 2-tert.butylglycine

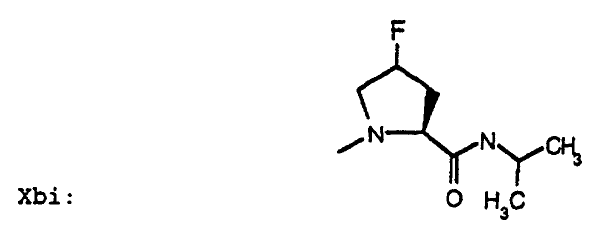

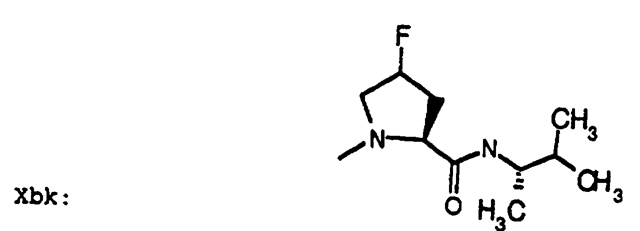

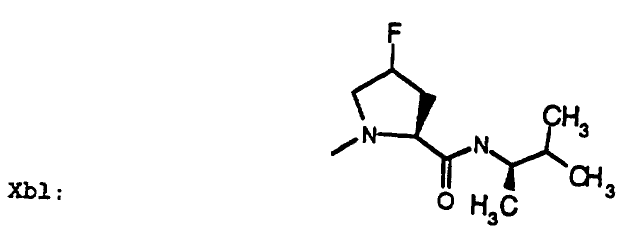

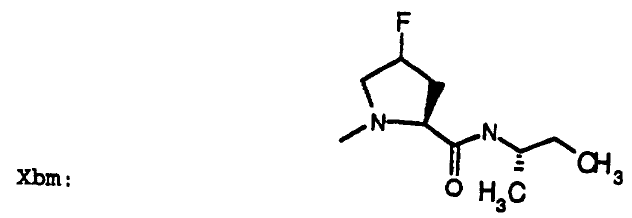

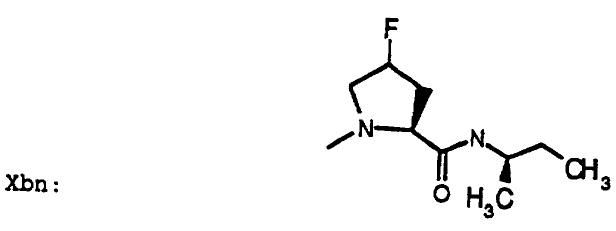

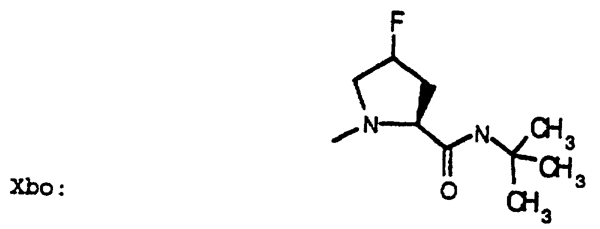

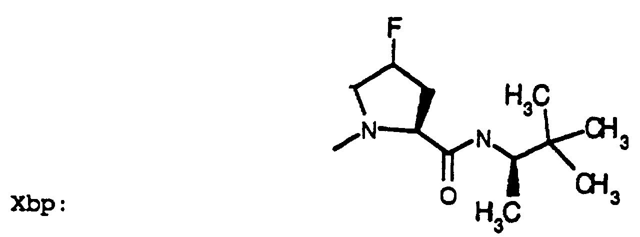

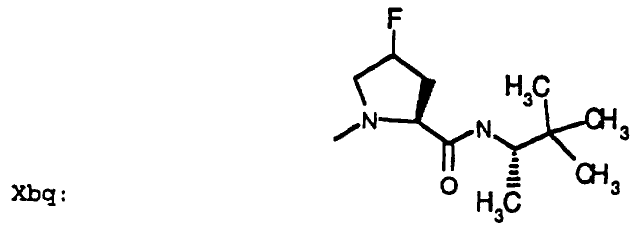

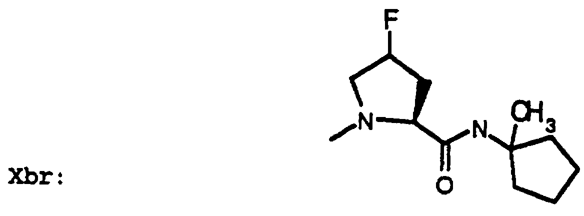

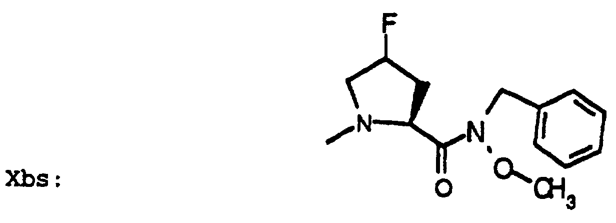

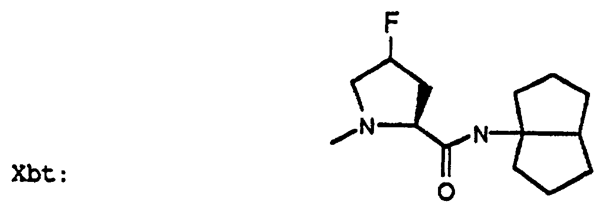

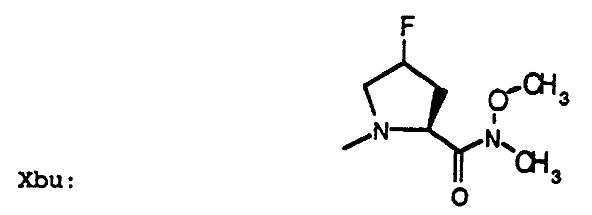

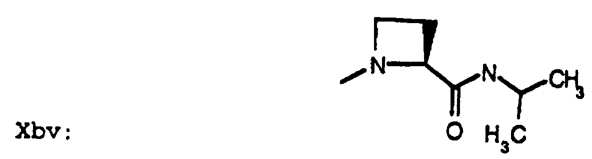

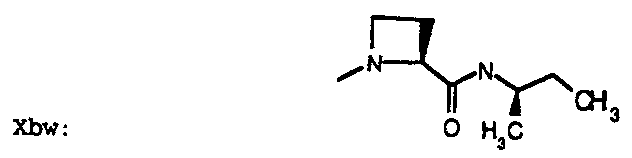

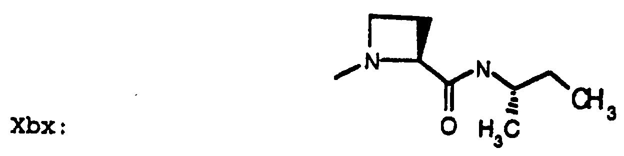

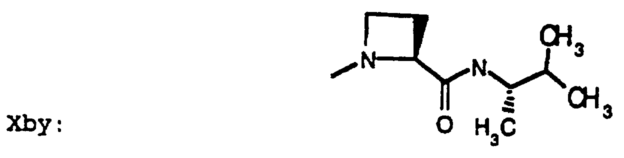

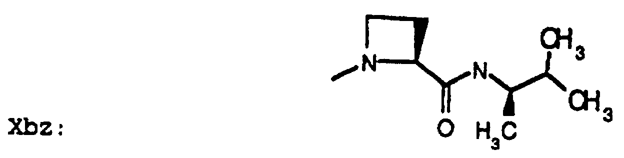

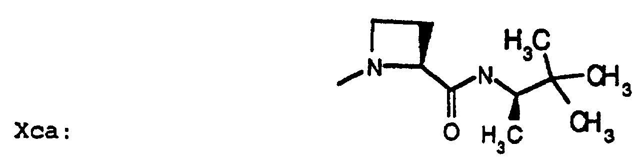

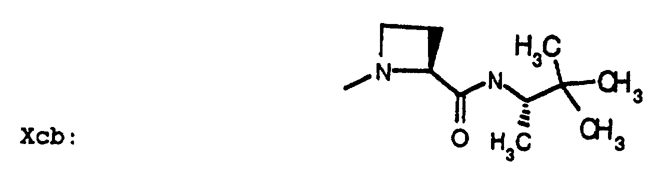

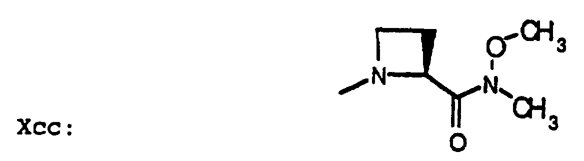

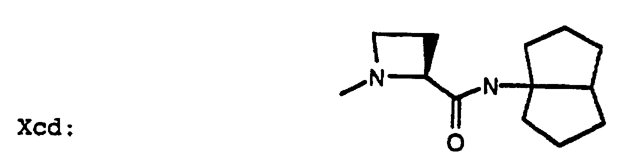

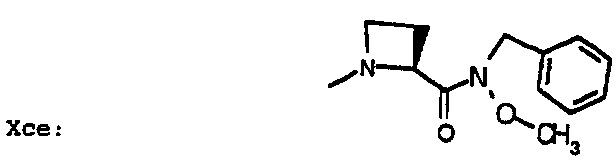

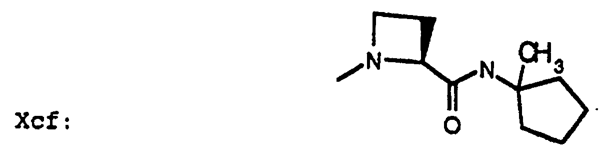

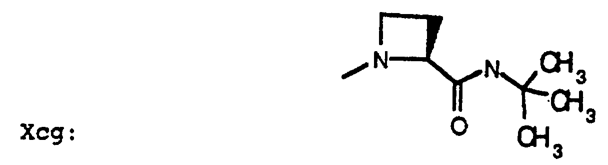

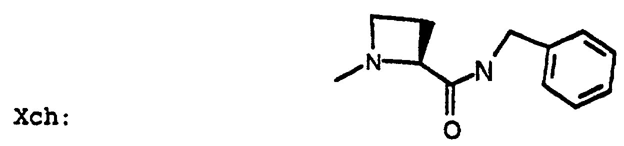

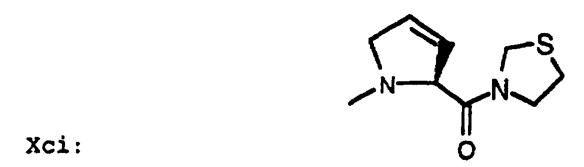

- Xck:: 3,4-dehydroproline-adamantyl(1)-amide

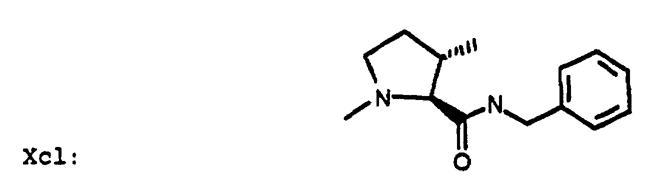

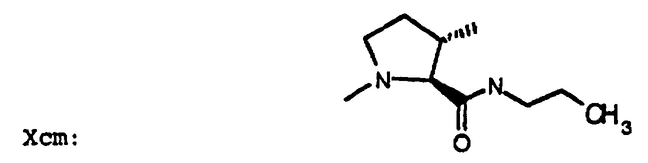

- Xcn:: 3,4-dehydroproline
- Xco:: 3,4-dehydroproline amide
- Xcp:: L-thiazolidine-4-carbonyl
- Xcq:: 4-fluoro-proline

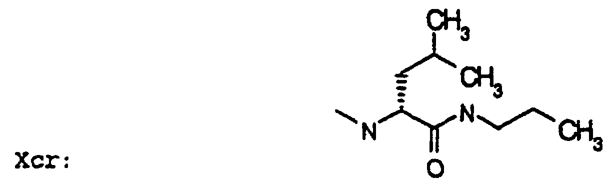

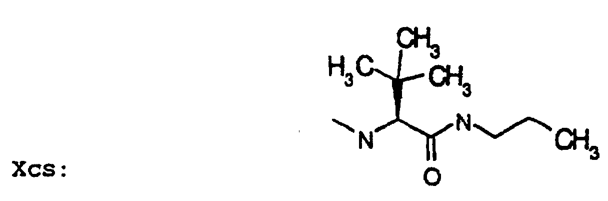

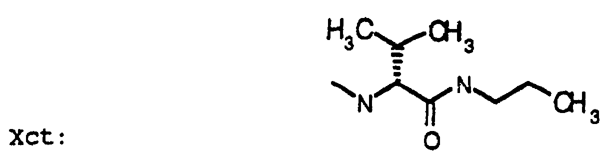

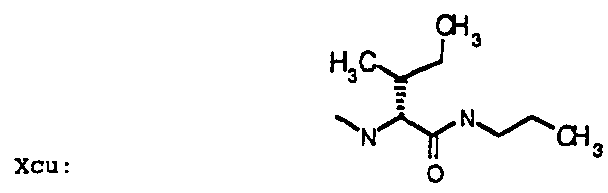

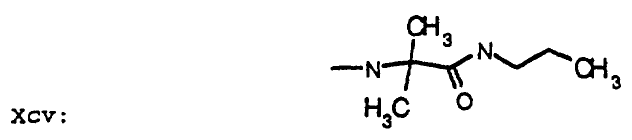

- Xcw:: L-azetidine-2-carbonyl

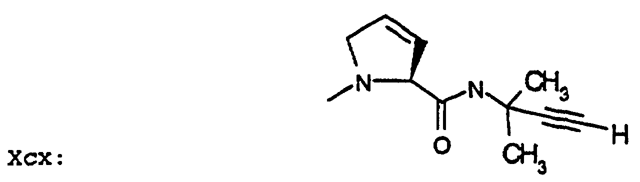

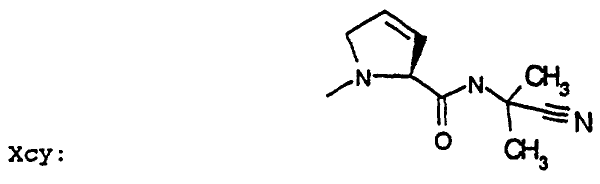

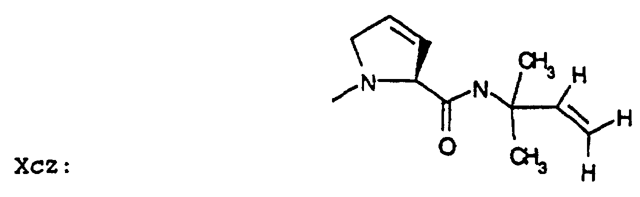

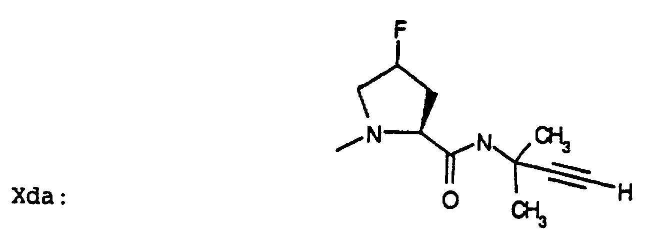

Compounds of this invention may be assayed for anti-cancer activity by conventional methods, including for example, the methods described below.

### A. In vitro methodology

Cytotoxicity was measured using a standard methodology for adherent cell lines such as the microculture tetrazolium assay (MTT). Details of this assay have been published (Alley, MC et al, Cancer Research 48:589-601, 1988). Exponentially growing cultures of tumor cells such as the HT-29 colon carcinoma or LX-l lung tumor are used to make microtiter plate cultures. Cells are seeded at 5000-20,000 cells per well in 96-well plates (in 150 µl of media), and grown overnight at 37°C. Test compounds are added, in 10-fold dilutions varying from 10⁻⁴ M to 10⁻¹⁰ M. Cells are then incubated for 48 hours. To determine the number of viable cells in each well, the MTT dye is added (50 µl of 3 mg/ml solution of 3-(4,5-dimethylthiazol-2-yl)-2,5-diphenyltetrazolium bromide in saline). This mixture is incubated at 37°C for 5 hours, and then 50 µl of 25 % SDS, pH2 is added to each well. After an overnight incubation, the absorbance of each well at 550 nm is read using an ELISA reader. The values for the mean +/- SD of data from replicated wells are calculated, using the formula % T/C (% viable cells treated/control).$\frac{\text{OD of treated cells}}{\text{OD of control cells}} \text{x 100 = % T/C}$

The concentration of test compound which gives a T/C of 50% growth inhibition was designated as the IC₅₀ value.

| COMPOUND OF EXAMPLE | IC₅₀ [M] |
|---|---|
| 1. | 6 X 10⁻⁶ |
| 2. | 1 x 10⁻⁹ |
| 61. | 2 x 10⁻⁸ |
| 64. | 2 x 10⁻⁸ |
| 88. | 4 x 10⁻⁸ |
| 89. | 6 x 10⁻⁸ |

### B. In vivo methodology

Compounds of this invention were further tested in preclinical assays for in vivo activity which is indicative of clinical utility. Such assays were conducted with nude mice into which tumor tissue, preferably of human origin, had been transplanted (xenografted), as is well known in this field. Test compounds were evaluated for their anti-tumor efficacy following administration to the xenograft-bearing mice.

More specifically, human breast tumors (MX-1) which had been grown in athymic nude mice were transplanted into new recipient mice, using tumor fragments which were about 50 mg in size. The day of transplantation was designated as day 0. Six to ten days later, mice were treated with the test compounds given as an intravenous injection, in groups of 5-10 mice at each dose. Compounds were given every other day, for 3 weeks, at doses from 1-100 mg/kg body weight. Tumor diameters and body weights were measured twice weekly. Tumor volumes were calculated using the diameters measured with Vernier calipers, and the formula${\text{(length x width}}^{\text{2}} {\text{)/2 = mm}}^{\text{3}} \text{of tumor volume}$

Mean tumor volumes are calculated for each treatment group, and T/C values determined for each group relative to the untreated control tumors.

The new compounds possess good tumor inhibiting properties.

## Claims

1. Peptides of the formula I
R¹R²N-CHX-CO-A-B-D-E-(F)ₜ-K I
where
R¹ is methyl, ethyl, or isopropylₓ;
R² is hydrogen, methyl, or ethyl;
R¹-N-R² together may be a pyrrollidine ring;
A is a valyl, isoleucyl, leucyl, 2-tert-butylglycyl, 2-ethylglycyl, norleucyl or norvalyl residue;
B is a N-methyl-valyl, -leucyl, -isoleucyl, -norvalyl, -norleucyl, -2-tert-butylglycyl, -3-tert-butylalanyl, or -2-ethylglycyl residue;
D is a prolyl, 3,4-dehydroprolyl, 4-fluoroprolyl, 4,4-difluoroprolyl, azetidine-2-carbonyl, homoprolyl, 3-methylprolyl, 4-methylprolyl, 5-methylprolyl, or thiazolidine-4-carbonyl residue;
E is a 3,4-dehydroprolyl, 4-fluoroprolyl, 3-methylprolyl, 4-methylprolyl, azetidine-2-carbonyl, or 4,4-difluoroprolyl residue;
F is a valyl, 2-tert-butylglycyl, isoleucyl, leucyl, 2-cyclohexylglycyl, norleucyl, norvalyl, neopentylglycyl, alanyl, β-alanyl, or aminoisobutyroyl residue;
X is alkyl (preferably C₂₋₅), cyclopropyl, or cyclopentyl;
t is 0 or 1; and
K is C₁₋₄-alkoxy, benzyloxy or an amino moiety of the formula R⁵-N-R⁶ wherein
R⁵ is hydrogen, or hydroxy, or C₁₋₄ alkoxy, or benzyloxy (which may be substituted by up to two substituents which may independently be CF₃, nitro, C₁₋₄ -alkylsulfonyl, C₁₋₄-alkoxy, halogen, C₁₋₄-alkyl, cyano, hydroxy, N(CH₃)₂, COOMe, COOEt, COOiPr, or CONH₂), or phenyloxy (which may be substituted by up to two substituents which may independently be CF₃, nitro, C₁₋₄-alkylsulfonyl, C₁₋₄-alkoxy, halogen, C₁₋₄-alkyl, cyano, hydroxy, N(CH₃)₂, COOMe, COOEt, COOiPr, or CONH₂);
R⁶ is hydrogen, or C₁₋₁₂-alkyl (which may be substituted by one or more fluoro atoms), or -C(CH₃)₂CN, or -C(CH₃)₂CCH, or -C(CH₃)₂C=CH₂, or -C(CH₃)₂CH₂OH, or -C(CH₃)₂CH₂CH₂OH, or -(CH₂)ᵥ-C₃₋₇-cycloalkyl (v=0,1,or 2) (which may be substituted by a methyl group), or norephedryl, or norpseudo-ephedryl, or quinolyl, or pyrazyl, or adamantyl, or -CH₂-benzimidazolyl, or -CH₂-adamantyl, or alpha-methyl-benzyl, or alpha-dimethylbenzyl, or -(CH₂)ᵥ-phenyl (v-0,1,2,or 3; which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or -(CH₂)ₘ-naphthyl (m=0 or 1; which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or -(CH₂)_{w}-benzhydryl (w=0,1, or 2; which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or
biphenyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or
pyridyl (which may be substituted by up to two substitutents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or picolyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
-CH₂-CH₂-pyridyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or benzothiazolyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or benzoisothiazolyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or benzopyrazolyl (which may be substituted by up to two substituents which may independently be CF₃, nitro, C₁₋₇-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or benzoxazolyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or -(CH₂)ₘ-fluorenyl (m=0 or 1; which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or pyrimidyl (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or -(CH₂)ₘ-indanyl (m=0 or 1; which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, N(CH₃)₂, COOMe, COOEt, COOiPr, or CONH₂), or -(CH₂CH₂O)_{y}-CH₃ (y=0,1,2,3,4, or 5), or -(CH₂CH₂O)_{y}-CH₂CH₃ (y=0,1,2,3,4, or 5), or -NH-C₆H₅ (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
-NCH₃-C₆H₅ (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
-NS-CH₂-C₆H₅ (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
-NCH₃-CH₂-C₆H₅ (which may be substituted by up to two substituents which may independently be CF₃, nitro, C₁₋₄-alkylsulfonyl, C₁₋₄-alkoxy, halogen, C₁₋₄-alkyl which may form a cyclic system, cyano, hydroxy, COOMe, COOEt, COOiPr, or CONH₂), or
5-membered heteroaryl which may be substituted by up to two substituents which may independently be CF₃, nitro; C₁₋₄-alkylsulfonyl, C₁₋₄-alkoxy, thiomethyl, thioethyl, picolyl, acetyl, C₃₋₆-cycloalkyl, thiophenyl, -CH₂-COOEt, C₃₋₄-alkylen group forming a bicyclic system with the heterocycle, phenyl (which may be substituted by up to two substituents which may independently be nitro, CF₃, CN, halogen, or
C₁₋₄-alkyl), benzyl (which may be substituted by up to two substituents which may independently be nitro, CF₃, halogen, C₁₋₄-alkyl, C₁₋₄-alkylsulfonyl, cyano, hydroxy, C₁₋₄-dialkylamino), or
-CHR⁷-5-membered heteroaryl (which may be substituted by up to two substituents which may independently be CF₃, nitro, cyano, halogen, COOMe, COOEt, COOiPr, CONH₂, C₁₋₄-alkyl, C₁₋₄-alkoxy, phenyl, benzyl, naphthyl, or C₁₋₄-alkylsulfonyl [R⁷ - hydrogen, linear or branched C₁₋₅-alkyl, benzyl]),
wherein -CHR⁷-5-membered heteroaryl may for example be represented by one of the folloging residues: 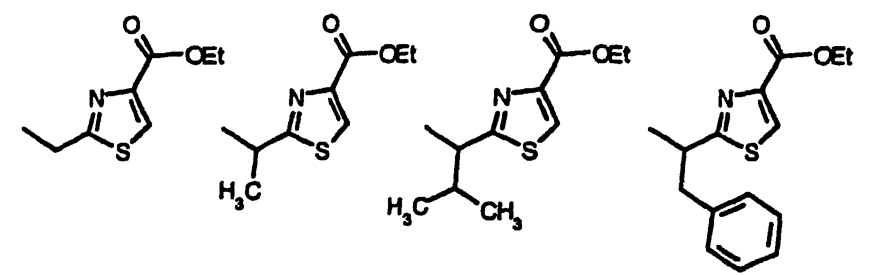 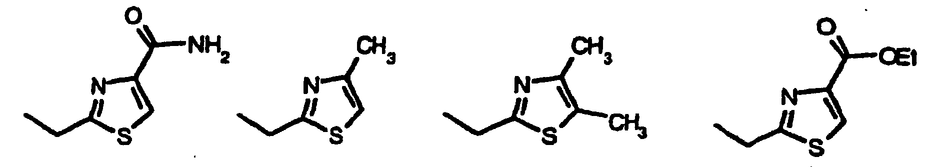 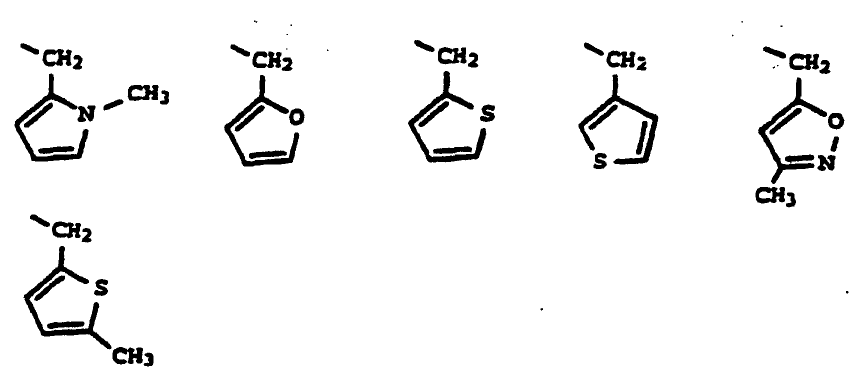 5-membered heteroaryl may for example be represented by the following residues: 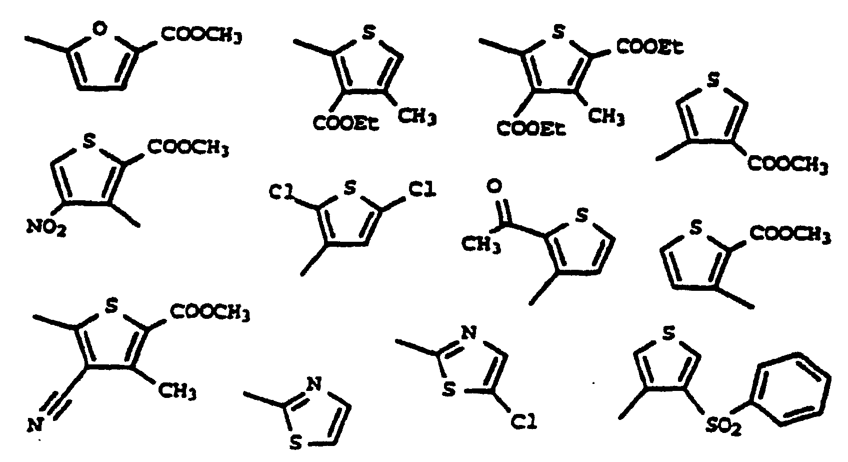 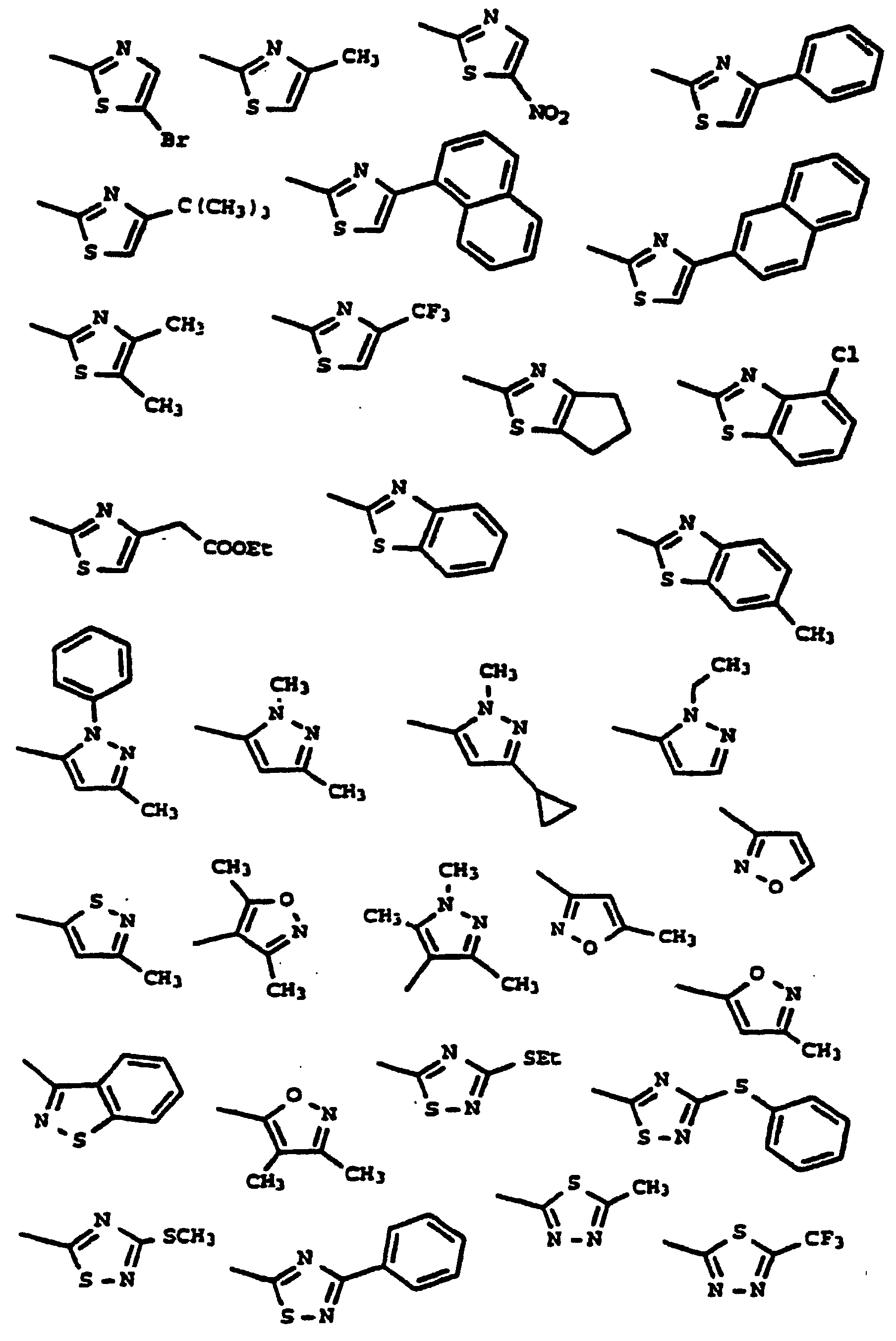 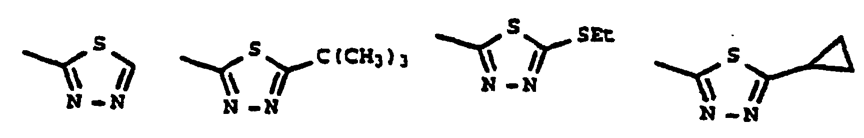 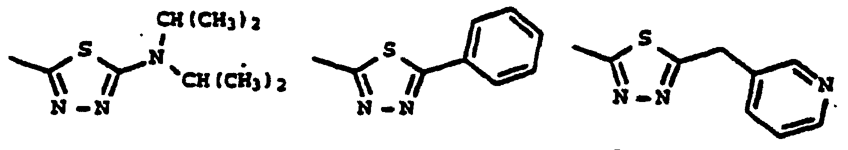 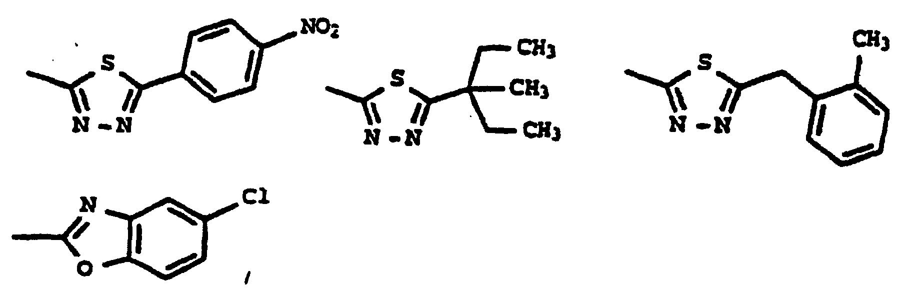 R⁵-H-R⁶ together may also form structures selected from the group consisting of 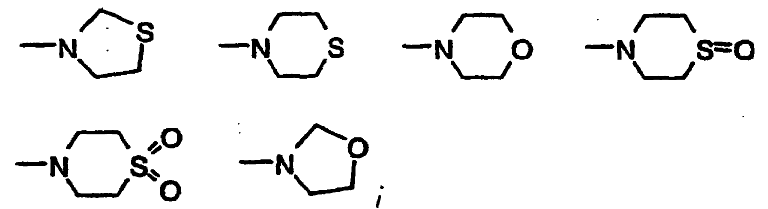 and the salts thereof with physiologically tolerated acids.

2. The compounds of claim 1, where
R¹ is methyl or ethyl;
R² is hydrogen, methyl, or ethyl;
A is a valyl, isoleucyl, 2-tert-butylglycyl residue;
B is a N-methyl-valyl, -isoleucyl, or -2-tert-butylglycyl residue;
D is a prolyl, 3,4-dehydroprolyl, 4-fluoroprolyl, 3-methylprolyl or thiazolidine-4-carbonyl residue;
E is a 3,4-dehydroprolyl, 4-fluoroprolyl, 3-methylprolyl, or azetidine-2-carbonyl residue;
F is a D-valyl, 2-tert-butylglycyl, D-isoleucyl, D-leucyl, or aminoisobutyroyl residue;
X is -CH(CH₃)₂, -C(CH₃)₃, or -CH(CH₃)CH₂CH₃;
t is 0 or 1 .

3. The compounds of claim 1 or claim 2, where K is
-OC(CH₃)₃, -NH₂, -NH-C₁₋₁₂-alkyl, -NH-C₃₋₈-cycloalkyl, -NH-[3,3,0]-bicyclooctyl, norephedryl, norpseudoephedryl, -NH-quinolyl, -NH-pyrazyl, -NH-CH₂-benzimidazolyl, -NH-adamantyl, -NH-CH₂-adamantyl, -NH-CH(CH₃)-phenyl, -NH-C(CH₃)₂-phenyl, -N(C₁₋₄-alkoxy)C₁₋₄-alkyl, -N(C₁₋₄-alkoxy)-CH₂-phenyl, -N(C₁₋₄-alkoxy)-phenyl, -N(CH₃)OBzl, -NH-(CH₂)ᵥ-phenyl (v=0,1,2,or 3), -NH-(CH₂)ₘ-naphthyl (m=0 or 1), -NH-(CH₂)_{w}-benzhydryl (w=0,1, or 2), -NH-biphenyl, -NH-pyridyl, -NH-CH₂-pyridyl, -NH-CH₂-CH₂-pyridyl, -NH-benzothiazolyl, -NH-benzoisothiazolyl, -NH-benzopyrazolyl, -NH-beazoxazolyl, -NH-(CH₂)ₘ-fluorenyl (m=0 or 1), -NH-pyrimidyl, -NH-(CH₂)ₘ-indanyl (m=0 or 1), -NH-(CH₂CH₂O)_{y}-CH₃ (y=0,1,2,3,4, or 5), -NH-(CH₂CH₂O)_{y}-CH₂CH₃ (y=0,1,2,3,4, or 5), -NH-(5-membered heteroaryl), -NH-CHR⁷-5-membered heteroaryl [R⁷ = hydrogen, linear or branched C₂₋₅-alkyl, benzyl] or K is

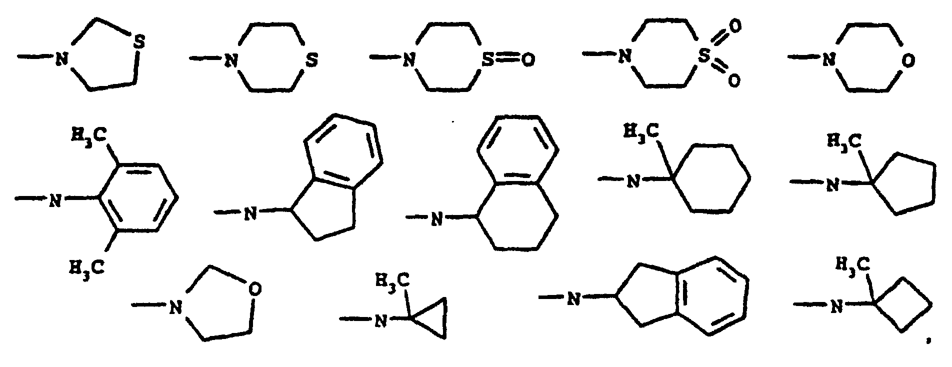

4. The compounds of any of claims 1 to 3, where K is
-NHCH₃, -NHCH₂CH₃, -NH(CH₂)₂CH₃, -NH(CH₂)₃CH₃, -NH(CH₂)₄CH₃, -NH(CH₂)₅CH₃, -NH(CH₂)₆CH₃, -NH(CH₂)₇CH₃, -NHCH(CH₃)₂, -NHCH(CH₃)CH₂CH₃, -NHCH(CH₃)CH₂CH₂CH₃, -NHCH(CH₂CH₃)₂, -NH(CH₂CH₂CH₃)₂, -NHC(CH₃)₃, -NHCH(CH₂CH₃)CH₂CH₂CH₃, -NHCH(CH₃)CH(CH₃)₂, -NHCH(CH₂CH₃)CH(CH₃)₂, -NHCH(CH₃)C(CH₃)₃, -NH-cyclopropyl, -NH-cyclobutyl, -NH-cyclopentyl, -NH-cyclohexyl,-NH-cycloheptyl, -NH-cyclooctyl, -NH-bicyclo[3,3,0]octyl, -N(CH₃)OCH₃, -N(CH₃)OCH₂CH₃, -N(CH₃)OCH₂CH₂CH₃, -N(CH₃)OCH(CH₃)₂, -N(CH₂CH₃)OCH₃, -N(CH₂CH₃)OCH₂CH₃ -N(CH(CH₃)₂)OCH₃, -N(CH₃)OCH₂C₆H₅, -N(OCH₃)CH₂-C₆H₅, -N(CH₃)OC₆H₅, -NH-CH₂-C₆H₅, -NH(CH₂)₂C₆H₅, -NH(CH₂)₃C₆H₅, -NHCH(CH₃)C₆H₅, -NHC(CH₃)₂C₆H₅, -NHC(CH₃)₂CH₂CH₃, -NHC(CH₃)(CH₂CH₃)₂, -NHCH(CH₃)CH(OH)C₆H₅, -NHCH₂-cyclohexyl, -NH-CH₂CF₃, -NHCH(CH₂F)₂, -NHC(CH₃)₂CH₂CH₂OH, -NH(CH₂CH₂O)₂CH₂CH₃, -NHC(CH₃)₂CH(CH₃)₂, -NHC(CH₃)₂CN, -NHC(CH₃)₂CCH, -NHC(CH₃)₂C=CH₂, norephedryl, norpseudoephedryl, -NH-quinolyl, -NH-pyrazyl, -NH-adamantyl(1), -NH-adamantyl(2), -NH-CH₂-adamantyl, -NH-CH₂-naphthyl, -NH-benzhydryl, -NH-biphenyl, -NH-pyridyl, -NH-picolyl, -NH-CH₂-CH₂-pyridyl, -NH-benzothiazolyl, -NH-benzoisothiazolyl, -NH-benzopyrazolyl, -NH-benzoxazolyl, -NH-fluorenyl, -NH-pyrimidyl, -NH-thiazolyl(2), -NH-isoxazolyl(3), -NH-CH₂-furanyl(2), -NH-(3-methyl)isoxazolyl(5), -NH-(3-methyl)isothiazolyl(5), -NH-(2-trifluormethyl)-thiadiazolyl(5), -OC(CH₃)₃, -NH-CH₂-(4-methyl)thiazolyl(2), -NH-CH₂-thienyl(2), -NH-CH₂-(5-methyl)thienyl(2), -NH-(2-methyl)-thiadiazolyl(5), -NH-(2-cyclopropyl)thiadiazolyl(5), or K is

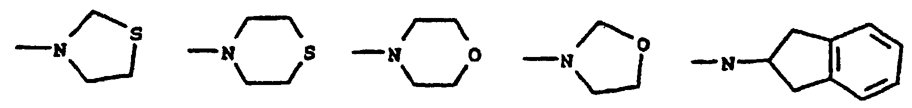

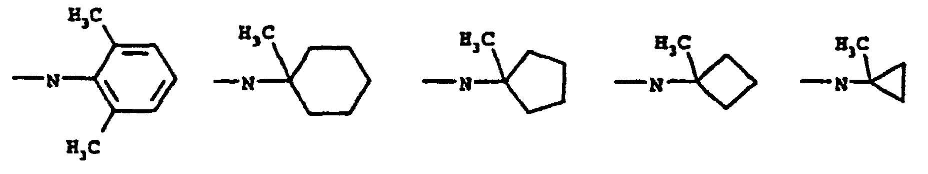

5. The compounds of any of claims 1 to 4 for use in medicine in particular for treating oncological diseases.

6. A pharmaceutical composition comprising a pharmaceutically acceptable carrier and a therapeutically effective amount of a compound of any of claims 1 to 4.

7. Use of a compound of any of claims 1 to 4 for preparing a pharmaceutical composition for treating a tumor in a mammal.

8. A method for preparing compounds of any of claims 1 to 4, **characterized in that** they are prepared according to known methods of peptide chemistry.

## Patentansprüche

1. Peptide der Formel I
R¹R²N-CHX-CO-A-B-D-E-(F)ₜ-K I
worin
R¹ für Methyl, Ethyl oder Isopropyl steht;
R² für Wasserstoff, Methyl oder Ethyl steht;
R¹-N-R² zusammen einen Pyrrolidin-Ring bilden können;
A für einen Valyl-, Isoleucyl-, Leucyl, 2-tert-Butylglycyl-, 2-Ethylglycyl-, Norleucyl- oder Norvalyl-Rest steht;
B für einen N-Methyl-valyl-, -leucyl-, -isoleucyl-, -norvalyl-, -norleucyl-, -2-tert-butylglycyl-, -3-tert-butylalanyl- oder -2-ethylglycyl-Rest steht;
D für einen Prolyl-, 3,4-Dehydroprolyl-, 4-Fluorprolyl-, 4,4-Difluorprolyl-, Azetidin-2-carbonyl-, Homoprolyl-, 3-Methylprolyl-, 4-Methylprolyl-, 5-Methylprolyl- oder Thiazolidin-4-carbonyl-Rest steht;
E für einen 3,4-Dehydroprolyl-, 4-Fluorprolyl-, 3-Methylprolyl-, 4-Methylprolyl-, Azetidin-2-carbonyl- oder einen 4,4-Difluoroprolyl-Rest steht;
F für einen Valyl-, 2-tert-Butylglycyl-, Isoleucyl-, Leucyl-, 2-Cyclohexylglycyl-, Norleucyl-, Norvalyl-, Neopentylglycyl-, Alanyl-, β-Alanyl- oder Aminoisobutyroyl-Rest steht;
X für Alkyl (vorzugsweise C₂₋₅), Cyclopropyl oder Cyclopentyl steht;
t 0 oder 1 ist; und
K für C₁₋₄-Alkoxy, Benzyloxy oder eine Aminoeinheit der Formel R⁵-N-R⁶ steht, worin
R⁵ für Wasserstoff oder Hydroxy oder C₁₋₄-Alkoxy oder Benzyloxy (welches durch bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, Cyano, Hydroxy, N(CH₃)₂, COOMe, COOEt, COOiPr, oder CONH₂ sein können), oder Phenyloxy (das substituiert sein kann mit bis zu zwei Substituenten, die unabhängig voneinander CF₃, Nitro, C₁₋₄-Alkylsulfonyl, C₁₋₄-Alkoxy, Halogen, C₁₋₄-Alkyl, Cyano, Hydroxy, N(CH₃)₂, COOMe, COOEt, COOiPr, oder CONH₂ sein können) steht;
R⁶ für Wasserstoff oder C₁₋₁₂-Alkyl (das mit einem oder mehreren Fluoratomen substituiert sein kann) oder -C(CH₃) ₂CN oder -C(CH₃) ₂CCH oder -C(CH₃)₂C=CH₂ oder -C(CH₃) ₂CH₂OH oder -C(CH₃) ₂CH₂CH₂OH oder -(CH₂)ᵥ-C₃₋₇-Cycloaklyl (v=0,1 oder 2) (das mit einer Methylgruppe substituiert sein kann) oder Norephedryl oder Norpseudoephedryl oder Chinolyl oder Pyrazyl oder Adamantyl oder -CH₂-Benzimidazolyl, oder -CH₂-Adamantyl oder Alpha-Methylbenzyl oder Alpha-Dimethylbenzyl oder -(CH₂)ᵥ-Phenyl (v = 0,1, 2 oder 3; das mit bis zu zwei Substituenten substituiert sein kann, die unabhängig voneinander CF_{3,} Nitro, C₁₋₄-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder -(CH₂)ₘ-Naphtyl (m = 0 oder 1; das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder -(CH₂)_{w}-Benzhydryl (w = 0,1 oder 2; das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder Biphenyl (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder Pyridyl (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, COOMe, COOEt, COOiPr, oder CONH₂ sein können) oder Picolyl (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, COOMe, COOEt, COOiPr, oder CONH₂ sein können) oder -CH₂-CH₂-Pyridyl (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, COOMe, COOEt, COOiPr, oder CONH₂ sein können) oder Benzothiazolyl (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder Benzoisothiazolyl (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder Benzopyrazolyl (das mit bis zu zwei Substituenten substituiert sein kann, die unabhängig voneinander CF₃, Nitro, C₁₋₇-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder Benzoxazolyl (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder -(CH₂)ₘ-Fluorenyl (m = 0 oder 1; das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder Pyrimidyl (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder -(CH₂)ₘ-Indanyl (m = 0 oder 1; das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, N(CH₃)₂, COOMe, COOEt, COOiPr, oder CONH₂ sein können) oder -(CH₂CH₂O)_{y}-CH₃ (y = 0,1, 2, 3, 4 oder 5) oder -(CH₂CH₂O)_{y}-CH₂CH₃ (y = 0,1, 2, 3, 4 oder 5) oder -NH-C₆H₅ (das mit bis zu zwei Substituenten substituiert sein kann, die unabhängig voneinander CF₃; Nitro, C₁₋₄-Alkylsulfonyl, C₁₋₄-Alkoxy, Halogen, C₁₋₄-Alkyl, das ein cyclisches System bilden kann, Cyano, Hydroxy, COOMe, COOEt, COOiPr, oder CONH₂ sein können) oder -NCH₃-C₆H₅ (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, COOMe, COOEt, COOiPr, oder CONH₂ sein können) oder -NH-CH₂-C₆H₅ (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, COOMe, COOEt, COOiPr, oder CONH₂ sein können) oder NCH₃-CH₂-C₆H₅ (das mit bis zu zwei Substituenten substituiert sein kann, die unabhängig voneinander CF₃, Nitro, C₁₋₄-Alkylsulfonyl, C₁₋₄-Alkoxy, Halogen, C₁₋₄-Alkyl, das ein cyclisches System bilden kann, Cyano, Hydroxy, COOMe, COOEt, COOiPr, oder CONH₂ sein können) oder 5-gliedriges Heteroaryl, das mit bis zu zwei Substituenten substituiert sein kann, die unabhängig voneinander CF₃, Nitro, C₁₋₄ Alkylsulfonyl, C₁₋₄-Alkoxy, Thiomethyl, Thioethyl, Picolyl, Acetyl, C₃₋₆-Cycloalkyl, Thiophenyl, -CH₂-COOEt, eine mit dem Heterocyclus ein bicyclisches System bildende C₃₋₄-Alkylengruppe, Phenyl (das mit bis zu zwei Substituenten substituiert sein kann, die unabhängig voneinander Nitro, CF₃, CN, Halogen oder C₁₋₄-Alkyl sein können), Benzyl (das mit bis zu zwei Substituenten substituiert sein kann, die unabhängig voneinander Nitro, CF₃, Halogen, C₁₋₄-Alkyl, C₁₋₄-Alkylsulfonyl, Cyano, Hydroxy, C₁₋₄-Dialkylamino sein können) sein können oder -CHR⁷-5-gliedriges Heteroaryl (das mit bis zu zwei Substituenten substituiert sein kann, die unabhängig voneinander CF₃, Nitro, Cyano, Halogen, COOMe, COOEt, COOiPr, CONH₂, C₁₋₄-Alkyl, C₁₋₄-Alkoxy, Phenyl, Benzyl, Naphtyl oder C₁₋₄-Alkylsulfonyl [R⁷ = Wasserstoff, lineares oder verzweigtes C₁₋₅-Alkyl, Benzyl] sein können), worin -CHR⁷-5-gliedriges Hereroaryl beispielsweise für einen der folgenden Reste stehen kann: 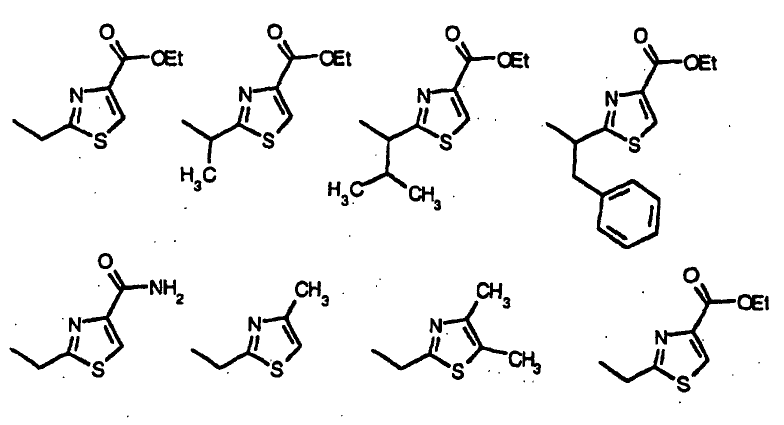 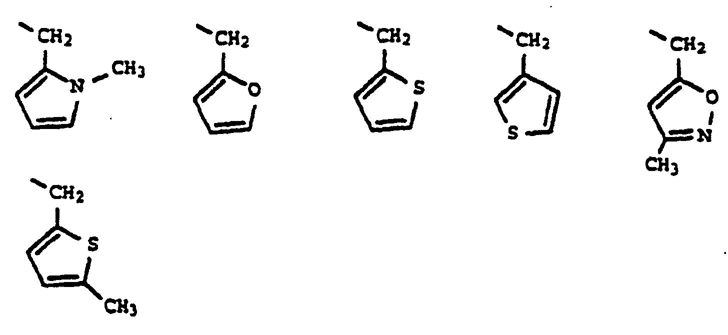 5-gliedriges Heteroaryl beispielsweise für die folgenden Reste stehen kann: 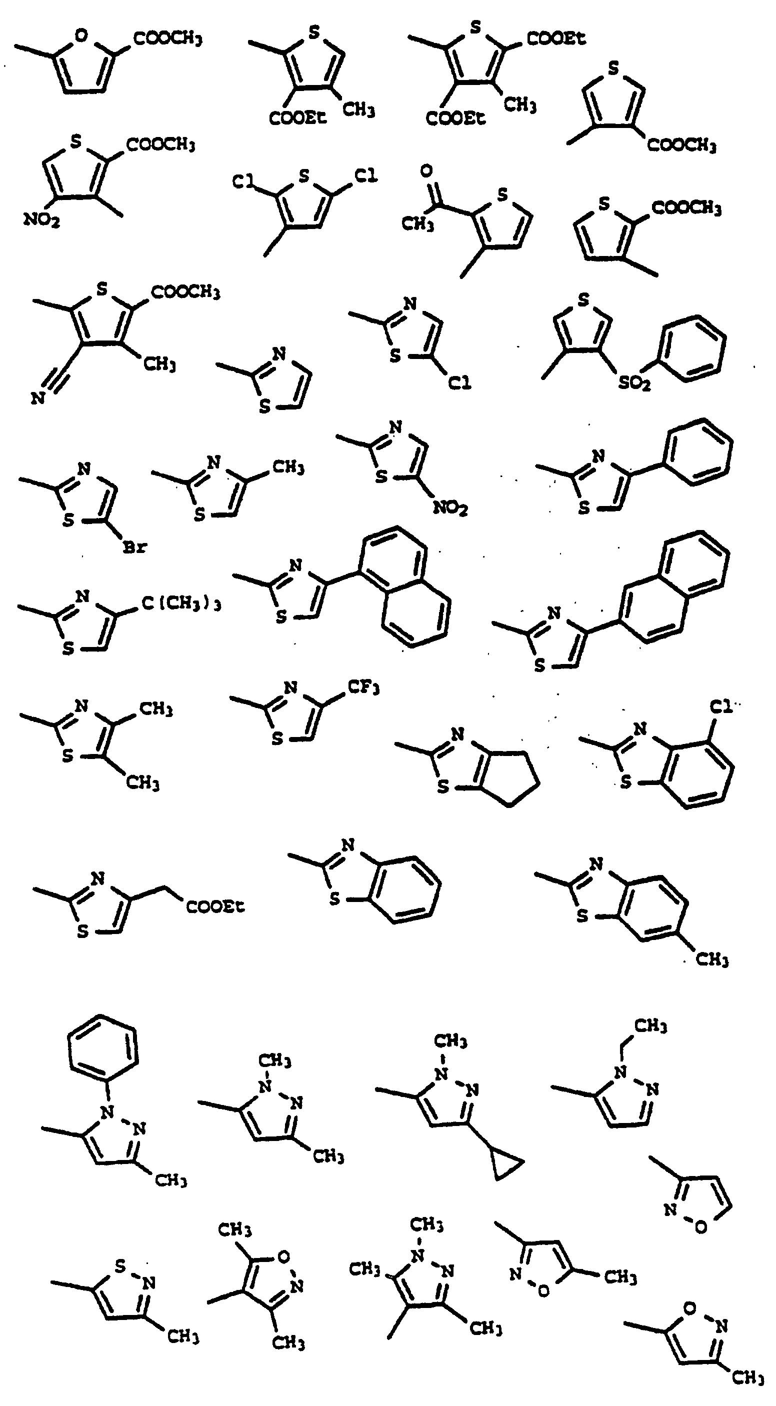 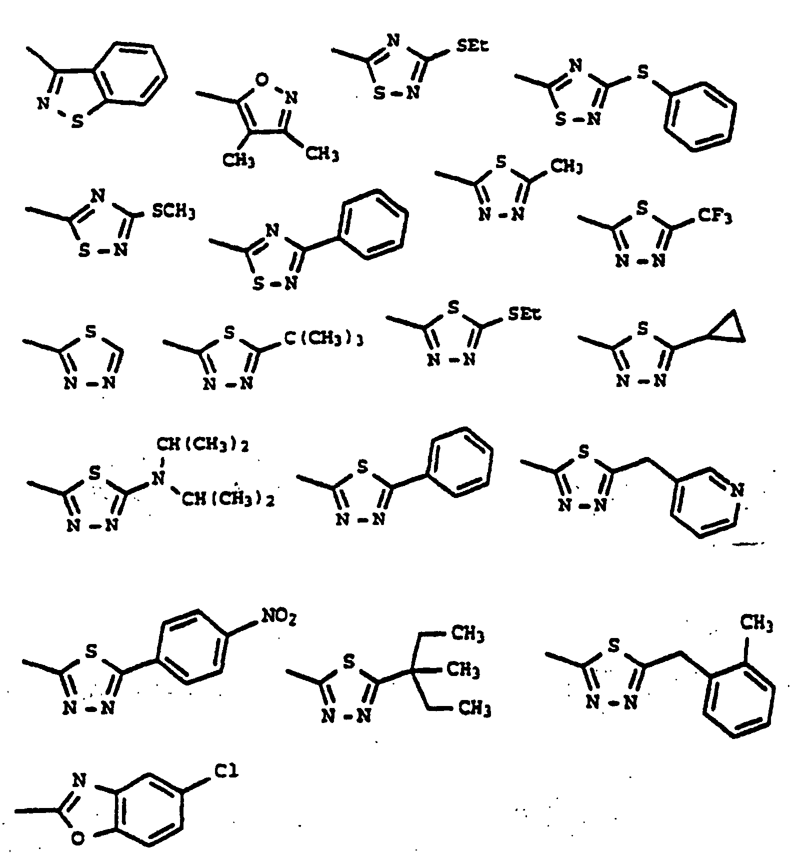 R⁵-N-R⁶ zusammen auch Strukturen bilden können, die ausgewählt sind unter 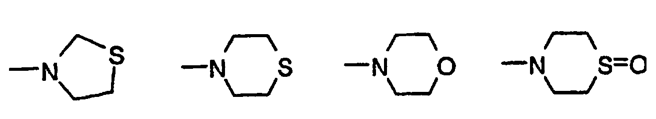 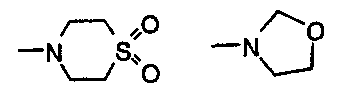 und Salze davon mit physiologisch verträglichen Säuren.

2. Verbindungen nach Anspruch 1, worin
R¹ Methyl oder Ethyl ist;
R² Wasserstoff, Methyl oder Ethyl ist;
A ein Valyl-, Isoleucyl-, 2-tert-Butylglycyl-Rest ist;
B ein N-Methyl-valyl-, -isoleucyl-, oder -2-tert-butylglycyl-Rest ist;
D ein Prolyl-, 3,4-Dehydroprolyl-, 4-Fluorprolyl-, 3-Methylprolyl- oder Thiazolidin-4-carbonyl-Rest ist;
E ein 3,4-Dehydroprolyl-, 4-Fluorprolyl-, 3-Methylprolyl- oder Azetidiri-2-carbonyl-Rest ist;
F ein D-Valyl-, 2-tert-Butylglycyl-, D-Isoleucyl-, D-Leucyl- oder Aminoisobutyroyl-Rest ist;
X -CH(CH₃)₂, -C(CH₃)₃ oder -CH(CH₃)CH₂CH₃ ist;
t 0 oder 1 ist.

3. Verbindung nach Anspruch 1 oder 2, worin K für
-OC(CH₃)₃, -NH₂, -NH-C₁₋₁₂-Alkyl, -NH-C₃₋₈-Cycloalkyl, -NH- [3,3,0]-Bicyclooctyl, Norephedryl, Norpseudoephedryl, -NH-Chinolyl, -NH-Pyrazyl, -NH-CH₂-Benzimidazolyl, -NH-Adamantyl, -NH-CH₂-Adamantyl, -NH-CH(CH₃)-Phenyl, -NH-C(CH₃)₂-Phenyl,-N(C₁₋₄-Alkoxy)-C₁₋₄-alkyl,-N(C₁₋₄-Alkoxy)-CH₂-phenyl, -N(C₁₋₄-Alkoxy)-phenyl, -N(CH₃)OBzl, -NH-(CH₂)ᵥ-Phenyl (v = 0, 1, 2, oder 3), -NH-(CH₂)ₘ-Naphthyl (m = 0 oder 1), -NH-(CH₂)_{w}-Benzhydryl (w = 0, 1, oder 2), -NH-Biphenyl, -NH-Pyridyl, -NH-CH₂-Pyridyl, -NH-CH₂-CH₂-Pyridyl, -NH-Benzothiazolyl, -NH-Benzothiazolyl -NH-Benzopyrazolyl, -NH-Benzoxazolyl, -NH-(CH₂)ₘ-Fluorenyl (m = 0 oder 1), -NH-Pyrimidyl, -NH-(CH₂)ₘ-Indanyl (m = 0 oder 1), -NH-(CH₂CH₂O)_{y}-CH₃ (y = 0, 1, 2, 3, 4, oder 5), -NH-(CH₂CH₂O)_{y}-CH₂CH₃ (y = 0, 1, 2, 3, 4, oder 5), -NH-(5-gliedriges Heteroaryl), -NH-CR⁷-5-gliedriges Heteroaryl [R⁷ = Wasserstoff, lineares oder verzweigtes C₁₋₅-Alkyl, Benzyl]
steht oder K 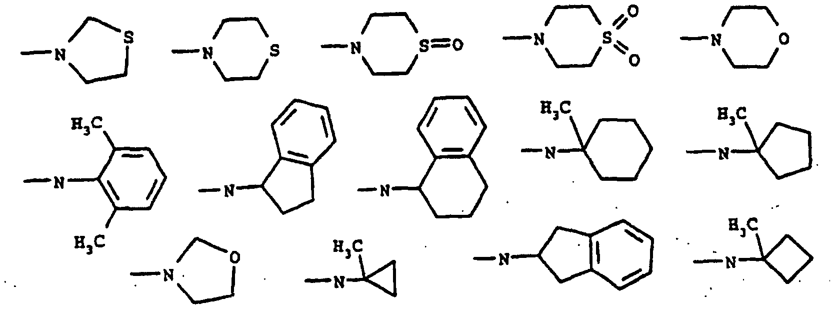 ist.

4. Verbindungen nach einem der Ansprüche 1 bis 3, worin K für
-NHCH₃, -NHCH₂CH₃, -NH(CH₂)₂CH₃, -NH(CH₂)₃CH₃, -NH(CH₂)₄CH₃, -NH(CH₂)₅CH₃,-NH(CH₂)₆CH₃, -NH(CH₂)₇CH₃, -NHCH(CH₃)₂, -NHCH(CH₃)CH₂CH₃, -NHCH(CH₃)CH₂CH₂CH₃, -NHCH(CH₂CH₃)₂, -NH(CH₂CH₂CH₃)₂, -NHC(CH₃)₃, -NHCH(CH₂CH₃)CH₂CH₂CH₃, -NHCH(CH₃)CH (CH₃)₂, -NHCH(CH₂CH₃)CH(CH₃)₂, -NHCH(CH₃)C(CH₃)₃, -NH-Cyclopropyl, -NH-Cyclobutyl, -NH-Cyclopentyl, -NH-Cyclohexyl, -NH-Cycloheptyl, -NH-Cyclooctyl, -NH-Bicyclo[3,3,0]octyl, N(CH₃)OCH₃, -N(CH₃)OCH₂CH₃, -N(CH₃)OCH₂CH₂CH₃, -N(CH₃)OCH(CH₃)₂, -N(CH₂CH₃)OCH₃, -N(CH₂CH₃)OCH₂CH₃, -N(CH(CH₃)₂)OCH₃, -N(CH₃)OCH₂C₆H₅, -N(OCH₃)CH₂-C₆H₅, -N(CH₃)OC₆H₅, -NH-CH₂-C₆H₅, -NH(CH₂)₂C₆H₅, -NH(CH₂)₃C₆H₅, -NHCH(CH₃)C₆H₅, -NHC(CH₃)₂C₆H₅, -NHC(CH₃)₂CH₂CH₃, -NHC(CH₃) (CH₂CH₃)₂, -NHCH(CH₃)CH(OH)C₆H₅, -NHCH₂-Cyclohexyl, -NH-CH₂CF₃, -NHCH(CH₂F)₂, -NHC(CH₃)₂CH₂CH₂OH, -NH(CH₂CH₂O)₂CH₂CH₃, -NHC(CH₃)₂CH(CH₃)₂, -NHC(CH₃)₂CN, -NHC(CH₃)₂CCH, -NHC(CH₃)₂C=CH_{2,} Norephedryl, Norpseudoephedryl, -NH-Chinolyl, -NH-Pyrazyl, -NH-Adamantyl(1), -NH-Adamantyl(2), -NH-CH₂-Adamantyl, -NH-CH₂-Naphthyl, -NH-Benzhydryl, -NH-Biphenyl, -NH-Pyridyl, -NH-Picolyl, -NH-CH₂CH₂-Pyridyl, -NH-Benzothiazolyl, -NH-Benzoisothiazolyl, -NH-Benzopyrazolyl, -NH-Benzoxazolyl, -NH-Fluorenyl, -NH-Pyrimidyl, -NH-Thiazolyl(2), -NH-Isoxazolyl(3), -NH-CH₂-Furanyl(2), -NH-(3-Methyl)isoxazolyl(5), -NH-(3-Methyl)isothiazolyl(5), -NH-(2-Trifluormethyl)-thiadiazolyl(5), OC(CH₃)₃, -NH-CH₂-(4-Methyl)thiazolyl(2),-NH-CH₂-Thienyl(2), -NH-CH₂-(5-Methyl)thienyl(2), -NH-(2-Methyl)thiadiazolyl(5), -NH-(2-Cyclopropyl)thiadiazolyl(5)
steht oder K 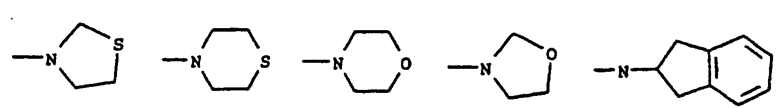 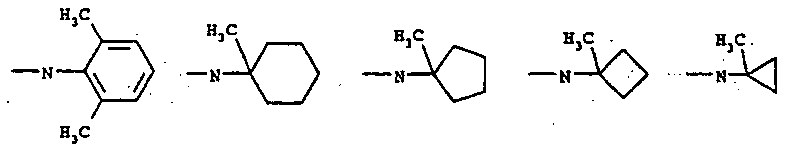 ist.

5. Verbindung nach einem der Ansprüche 1 bis 4 zur medizinischen Verwendung insbesondere für die Behandlung onkologischer Erkrankungen.

6. Pharmazeutische Zusammensetzung, umfassend ein pharmazeutisch verträglichen Träger und eine therapeutisch wirksame Menge einer Verbindung nach einem der Ansprüche 1 bis 4.

7. Verwendung einer Verbindung nach einem der Ansprüche 1 bis 4 zur Herstellung einer pharmazeutischen Zusammensetzung zur Behandlung eines Tumors in einem Säuger.

8. Verfahren zur Herstellung von Verbindungen nach einem der Ansprüche 1 bis 4, **dadurch gekennzeichnet, dass** man sie in Anlehnung an bekannte peptidchemische Verfahren herstellt.

## Revendications

1. Peptides de formule I
R¹R²N-CHX-CO-A-B-D-E-(F)ₜ-K I
où
R¹ est un groupe méthyle, éthyle ou isopropyle ;
R² est l'hydrogène, un groupe méthyle ou éthyle ;
R¹-N-R² ensemble peuvent former un cycle de pyrrolidine ;
A est un résidu valyle, isoleucyle, leucyle, 2-tert-butylglycyle, 2-éthylglycyle, norleucyle ou norvalyle ;
B est un résidu N-méthyl-valyle, -leucyle, -isoleucyle, -norvalyle, -norleucyle, -2-tert-butylglycyle, -3-tert-butylalanyle ou -2-éthylglycyle ;
D est un résidu prolyle, 3,4-déshydroprolyle, 4-fluoroprolyle, 4,4-difluoroprolyle, azétidine-2-carbonyle, homoprolyle, 3-méthylprolyle, 4-méthylprolyle, 5-méthylprolyle ou thiazolidine-4-carbonyle ;
E est un résidu 3,4-déshydroprolyle, 4-fluroprolyle, 3-méthylprolyle, 4-méthylprolyle, azétidine-2-carbonyle ou 4,4-difluoroprolyle ;
F est un résidu valyle, 2-tert-butylglycyle, isoleucyle, leucyle, 2-cyclohexylglycyle, norleucyle, norvalyle, néopentylglycyle, alanyle, β-alanyle ou aminoisobutyroyle ;
X est un groupe alkyle (de préférence en C₂-C₅), cyclopropyle ou cyclopentyle ;
t est 0 ou 1 ; et
K est un groupe alcoxy en C₁-C₄, benzyloxy ou un fragment amino de formule R⁵-N-R⁶ où
R⁵ est l'hydrogène ou un groupe hydroxyle, ou alcoxy en C₁-C₄, ou benzyloxy (qui peut être substitué par jusqu'à deux substituants qui peuvent être indépendamment des groupes CF₃, nitro, alkylsulfonyle en C₁-C₄, alcoxy en C₁-C₄, halogène, alkyle en C₁-C₄, cyano, hydroxyle, N(CH₃)₂, COOMe, COOEt, COOiPr, ou CONH₂), ou phényloxy (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄, cyano, hydroxyle, N(CH₃)₂, COOMe, COOEt, COOiPr, ou CONH₂) ;
R⁶ est l'hydrogène, ou un groupe alkyle en C₁-C₁₂ (qui peut être substitué par un ou plusieurs atomes de fluor), ou -C(CH₃)₂CN, ou -C(CH₃)₂CCH ou -C(CH₃)₂C=CH₂, ou -C(CH₃)₂CH₂OH, ou -C(CH₃)₂CH₂CH₂OH, ou -(CH₂)ᵥ-cycloalkyle en C₃-C₇ (v = 0, 1 ou 2) (qui peut être substitué par un groupe méthyle), ou noréphédryle ou norpseudoéphédryle, ou quinolyle, ou pyrazyle, ou adamantyle, ou -CH₂-benzimidazolyle, ou -CH₂-adamantyle, ou alpha-méthylbenzyle, ou alpha-diméthylbenzyle, ou -(CH₂)ᵥ-phényle (v = 0, 1, 2 ou 3 ; qui peut être substitué par jusqu'à deux substituants qui. peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, N(CH₃)₂, COOMe, COOEt, COOiPr, ou CONH₂), ou -(CH₂)ₘ-naphtyle (m = 0 ou 1 ; qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, N(CH₃)₂, COOMe, COOEt, COOiPr ou CONH₂), ou -(CH₂)_{w}-benzhydryle (w = 0, 1 ou 2 ; qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, N(CH₃)₂, COOMe, COOEt, COOiPr ou CONH₂), ou
biphénylyle (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, N(CH₃)₂, COOMe, COOEt, COOiPr ou CONH₂), ou pyridyle (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, COOMe, COOEt, COOiPr ou CONH₂), ou
picolyle (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, COOMe, COOEt, COOiPr ou CONH₂), ou picolyle (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, COOMe, COOEt, COOiPr ou CONH₂), ou
-CH₂-CH₂-pyridyle (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, COOMe, COOEt, COOiPr ou CONH₂), ou
benzothiazolyle (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, N(CH₃)₂, COOMe, COOEt, COOiPr ou CONH₂), ou
benzoisothiazolyle (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, N(CH₃)₂, COOMe, COOEt, COOiPr ou CONH₂), ou
benzopyrazolyle (qui peut être substitué par jusqu'à deux substituants qui peuvent être indépendamment des groupes CF₃, nitro, (alkyle en C₁-C₇)sulfonyle, alcoxy en C₁-C₄, halogéno, alkyle en C₁-C₄ qui peut former un système cyclique, cyano, hydroxyle, N(CH₃)₂, COOMe, COOEt, COOiPr ou CONH₂), ou
benzoxazolyle (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, N(CH₃)₂, COOMe, COOEt, COOiPr ou CONH₂), ou
-(CH₂)ₘ-fluorényle (m = 0 ou 1 ; qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, N(CH₃)₂, COOMe, COOEt, COOiPr ou CONH₂), ou
pyrimidyle (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, N(CH₃)₂, COOMe, COOEt, COOiPr ou CONH₂), ou
-(CH₂)ₘ-indanyle (m = 0 ou 1 ; qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, N(CH₃)₂, COOMe, COOEt, COOiPr ou CONH₂), ou
-(CH₂CH₂O)_{y}-CH₃ (y = 0, 1, 2, 3, 4 ou 5), ou
-(CH₂CH₂O)_{y}-CH₂CH₃ (y = 0, 1, 2, 3, 4 ou 5), ou
-NH-C₆H₅ (qui peut être substitué par jusqu'à deux substituants qui peuvent être indépendamment des groupes CF_{3,} nitro, alkyisulfonyle en C₁-C₄, alcoxy en C₁-C₄, halogéno, alkyle en C₁-C₄ qui peut former un système cyclique, cyano, hydroxyle, COOMe, COOEt, COOiPr ou CONH₂), ou
-NCH₃-C₆H₅ (qui peut être substitué par jusqu'à deux substituants qui peuvent être indépendamment des groupes CF_{3,} nitro, alkylsulfonyle en C₁-C₄, alcoxy en C₁-C₄, halogéno, alkyle en C₁-C₄ qui peut former un système cyclique, cyano, hydroxyle, COOMe, COOEt, COOiPr ou CONH₂), ou
-NH-CH₂-C₆H₅ (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, COOMe, COOEt, COOiPr ou CONH₂), ou
-NCH₃-CH₂-C₆H₅ (qui peut être substitué par jusqu'à deux substituants qui peuvent être indépendamment des groupes CF₃, nitro, alkylsulfonyle en C₁-C₄, alcoxy en C₁-C₄, halogéno, alkyle en C₁-C₄ qui peut former un système cyclique, cyano, hydroxyle, COOMe, COOEt, COOiPr ou CONH₂), ou
hétéroaryle pentagonal qui peut être substitué par jusqu'à deux substituants qui peuvent être indépendamment des groupes CF₃, nitro, alkylsulfonyle en C₁-C₄, alcoxy en C₁-C₄, thiométhyle, thioéthyle, picolyle, acétyle, cycloalkyle en C₃-C₆, thiophényle, -CH₂-COOEt, alkylène en C₃-C₄ formant un système bicyclique avec l'hétérocycle, phényle (qui peut être substitué par jusqu'à deux substituants qui peuvent être indépendamment des groupes nitro, CF₃, CN, halogéno, ou alkyle en C₁-C₄), benzyle (qui peut être substitué par jusqu'à deux substituants qui peuvent être indépendamment des groupes nitro, CF₃, halogéno, alkyle en C₁-C₄, alkylsulfonyle en C₁-C₄, cyano, hydroxyle, dialkylamino en C₁-C₄), ou
-CHR⁷-hétéroaryle pentagonal (qui peut être substitué par jusqu'à deux substituants qui peuvent être indépendamment des groupes CF₃, nitro, cyano, halogéno, COOMe, COOEt, COOiPr, CONH₂, alkyle en C₁-C₄, alcoxy en C₁-C₄, phényle, benzyle, naphtyle, ou alkylsulfonyle en C₁-C₄ [R⁷ = hydrogène, groupe alkyle en C₁-C₅ linéaire ou ramifié, benzyle]),
où
le groupe -CHR⁷-hétéroaryle pentagonal peut être par exemple représenté par l'un des résidus suivants : 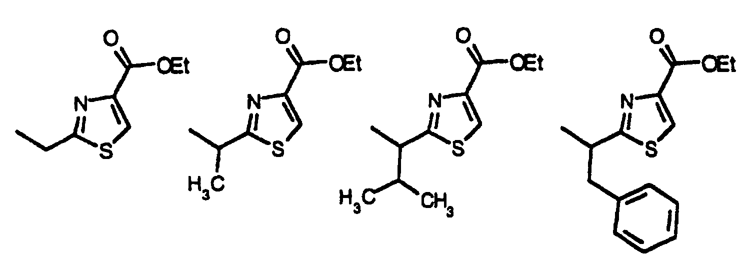 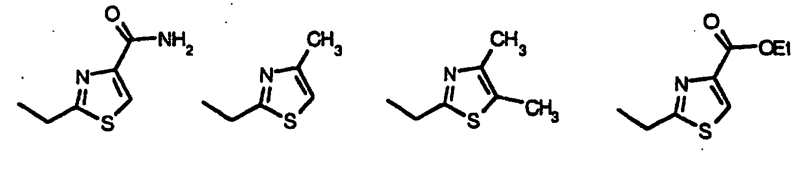 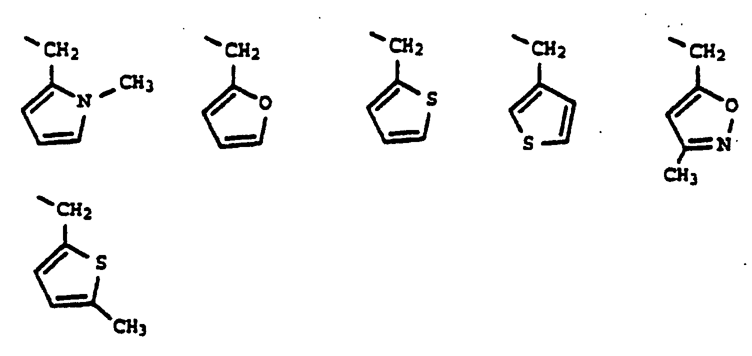 le groupe hétéroaryle pentagonal peut être par exemple représenté par les résidus suivants : 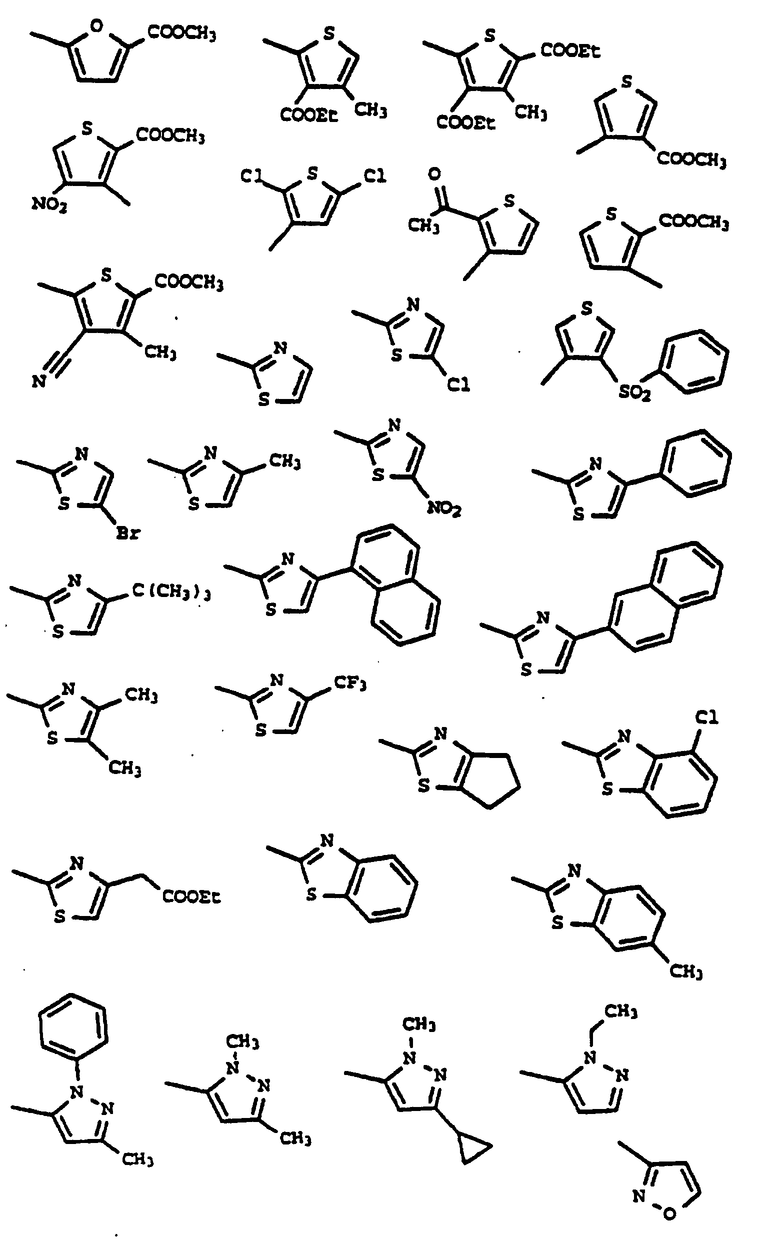 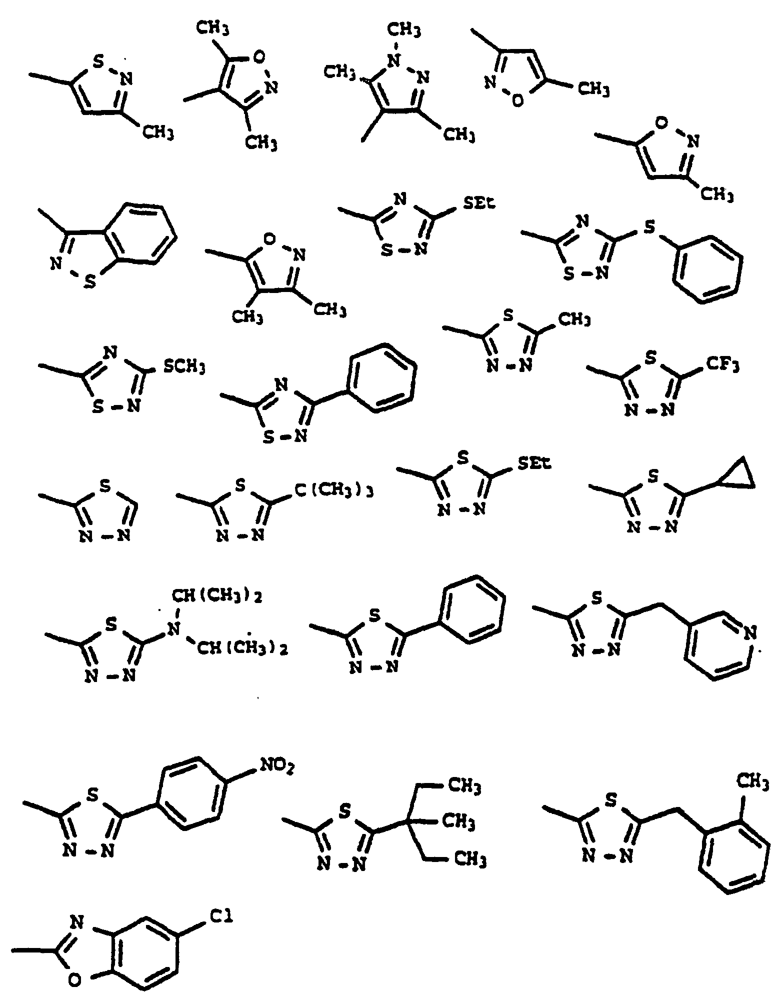 R⁵-N-R⁶ ensemble peuvent également former des structures choisies dans la classe formée par 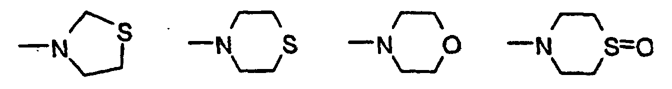 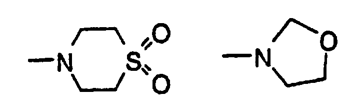 et leurs sels avec des acides physiologiquement tolérés.

2. Composés selon la revendication 1, dans lesquel
R¹ est un groupe méthyle ou éthyle ;
R² est l'hydrogène, un groupe méthyle ou éthyle ;
A est un résidu valyle, isoleucyle, 2-*tert*-butylglycyle ;
B est résidu N-méthyl-valyle, -isoleucyle, ou -2-*tert*-butylglycyle ;
D est un résidu prolyle, 3,4-déshydroprolyle, 4-fluoroxprolyle, 3-méthylprolyle ou thiazolidine-4-carbonyle ;
E est un résidu 3,4-déshydroprolyle, 4-fluoroprolyle, 3-méthylprolyle ou azétidine-2-carbonyle ;
F est un résidu D-valyle, 2-tert-butylglycyle, D-isoleucyle, D-leucyle ou aminoisobutyroyle ;
X est -CH(CH₃)₂, -C(CH₃)₃ ou -CH(CH₃)CH₂CH₃ ;
t est 0 ou 1.

3. Composés selon la revendication 1 ou la revendication 2, dans lesquels K est un groupe
-OC(CH₃)₃, -NH₂, -NH-alkyle en C₁-C₁₂, -NH-cycloalkyle en C₃-C₈, -NH-[3,3,0]-bicyclooctyle, noréphédryle, norpseudoéphédryle, -NH-quinolyle, -NH-pyrazyle, -NH-CH₂-benzimidazolyle, -NH-adamantyle, -NH-CH₂-adamantyle, -NH-CH(CH₃)-phényle, -NH-C(CH₃)₂-phényle, -N(alcoxy en C₁-C₄)-alkyle en C₁-C₄, -N(alcoxy en C₁-C₄)-CH₂-phényle, -N(alcoxy en C₁-C₄)-phényle, -N(CH₃)OBzl, -NH- (CH₂)ᵥ-phényle (v = 0, 1, 2 ou 3), -NH-(CH₂)ₘ-naphtyle (m = 0 ou 1), -NH-(CH₂)_{w}-benzhydryle (w = 0, 1 ou 2), -NH-biphénylyle, -NH-pyridyle, -NH-CH₂-pyridyle, -NH-CH₂-CH₂-pyridyle, -NH-benzothiazolyle, -NH-benzoisothiazolyle, -NH-benzopyrazolyle, -NH-benzoxazolyle, -NH-(CH₂)ₘ-fluorényle (m = 0 ou 1), -NH-pyrimidyle, -NH-(CH₂)ₘ-indanyle (m = 0 ou 1), -NH-(CH₂CH₂O)_{y}-CH₃ (y = 0, 1, 2, 3, 4 ou 5), -NH-(CH₂CH₂O)_{y}-CH₂CH₃ (y = 0, 1, 2, 3, 4 ou 5), -NH-hétéroaryle pentagonal, -NH-CHR⁷-hétéroaryle pentagonal [R⁷ = hydrogène, groupe alkyle en C₁-C₅ linéaire ou ramifié, benzyle] ; ou bien K est

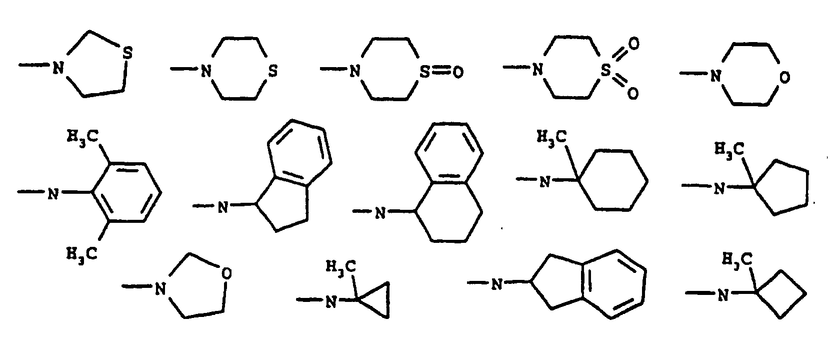

4. Composés selon l'une quelconque des revendications 1 à 3, dans lesquels K est
-NHCH₃, -NHCH₂CH₃, -NH(CH₂)₂CH₃, -NH(CH₂)₃CH₃, -NH(CH₂)₄CH₃, -NH(CH₂)₅CH₃, -NH(CH₂)₆CH₃, -NH(CH₂)₇CH₃, -NHCH(CH₃)₂, -NHCH(CH₃)CH₂CH₃, -NHCH(CH₃)CH₂CH₂CH₃, -NHCH(CH₂CH₃)₂, -NH(CH₂CH₂CH₃)₂, -NHC(CH₃)₃, -NHCH(CH₂CH₃)CH₂CH₂CH₃, -NHCH(CH₃)CH(CH₃)₂, -NHCH(CH₂CH₃)CH(CH₃)₂, -NHCH(CH₃)C(CH₃)₃, -NH-cyclopropyle, -NH-cyclobutyle, -NH-cyclopentyle, -NH-cyclohexyle, -NH-cycloheptyle, -NH-cyclooctyle, -NH-bicyclo[3,3,0]octyle, -N(CH₃)OCH₃, -N(CH₃)OCH₂CH₃, -N(CH₃)OCH₂CH₂CH₃, -N(CH₃)OCH(CH₃)₂, -N(CH₂CH₃)OCH₃, -N(CH₂CH₃)OCH₂CH₃, -N(CH(CH₃)₂)OCH₃, -N(CH₃)OCH₂C₆H₅, -N(OCH₃)CH₂-C₆H₅, -N(CH₃)OC₆H₅, -NH-CH₂-C₆H₅, -NH(CH₂)₂C₆H₅, -NH(CH₂)₃C₆H₅, -NHCH(CH₃)C₆H₅, -NHC(CH₃)₂C₆H₅, -NHC(CH₃)₂CH₂CH₃, -NHC(CH₃)(CH₂CH₃)₂, -NHCH(CH₃)CH(OH)C₆H₅, -NHCH₂-cyclohexyle, -NH-CH₂CF₃, -NHCH(CH₂F)₂, -NHC(CH₃)₂CH₂CH₂OH, -NH(CH₂CH₂O)₂CH₂CH₃, -NHC(CH₃)₂CH(CH₃)₂, -NHC(CH₃)₂CN, -NHC(CH₃)₂CCH, -NHC(CH₃)₂C=CH₂, noréphédryle, norpseudoéphédryle, -NH-quinolyle, -NH-pyrazyle, -NH-adamantyle (1), -NH-adamantyle(2), -NH-CH₂-adamantyle, -NH-CH₂-naphtyle, -NH-benzhydryle, -NH-biphénylyle, -NH-pyridyle, -NH-picolyle, -NH-CH₂-CH₂-pyridyle, -NH-benzothiazolyle, -NH-benzoisothiazolyle, -NH-benzopyrazolyle, -NH-benzoxazolyle, -NH-fluorényle, -NH-pyrimidyle, -NH-thiazolyle(2), -NH-isoxazolyle(3), -NH-CH₂-furannyle(2), -NH-(3-méthyl)isoxazolyle(5), -NH-(3-méthyl)isothiazolyle(5), -NH-(2-trifluorométhyl)-thiadiazolyle(5), -OC(CH₃)₃, -NH-CH₂-(4-méthyl)-thiazolyle(2), -NH-CH₂-thiényle(2), -NH-CH₂-(5-méthyl)-thiényle(2), -NH-(2-méthyl)thiadiazolyle(5), -NH-(2-cyclopropyl)thiadiazolyle(5), ou bien K est

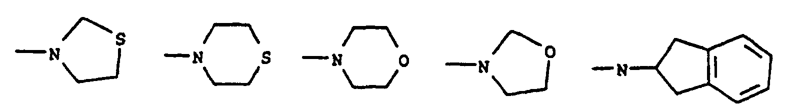

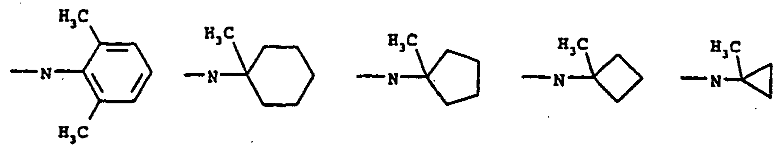

5. Composés selon l'une quelconque des revendications 1 à 4, pour leur utilisation en médecine, notamment pour traiter des maladies oncologiques.

6. Composition pharmaceutique comprenant un support pharmaceutiquement acceptable et une quantité thérapeutiquement efficace d'un composé selon l'une quelconque des revendications 1 à 4.

7. Utilisation d'un composé selon l'une quelconque des revendications 1 à 4, pour la préparation d'une composition pharmaceutique destinée à traiter une tumeur chez un mammifère.

8. Procédé pour la préparation des composés selon l'une quelconque des revendications 1 à 4, **caractérisé en ce qu'**ils sont préparés selon des procédés connus de la chimie des peptides.


### SEQUENCE LISTING

(1) GENERAL INFORMATION
   (i) APPLICANT:
      (A) BASF Aktiengesellschaft
      (B) STREET: Carl-Bosch-Strasse 38
      (C) CITY: Ludwigshafen
      (E) COUNTRY: Bundesrepublik Deutschland
      (F) ZIP: D-67056
      (G) TELEPHONE: 0621/6048526
      (H) TELEFAX: 0621/6043123
      (I) TELEX: 1762175170
   (ii) TITLE OF INVENTION: Novel peptides, the preparation and use thereof
   (iii) NUMBER OF SEQUENCES: 5
   (iv) COMPUTER READABLE FORM:
      (A) MEDIUM TYPE: Diskette, 3,5 inch, 2 DD
      (B) COMPUTER: IBM AT-compatible, 80286 processor
      (C) OPERATING SYSTEM: MS-DOS version 5.0
      (D) SOFTWARE: WordPerfect
(2) INFORMATION FOR SEQ ID NO: 1:
   (i) SEQUENCE CHARACTERISTICS:
      (A) LENGTH: 5 amino acids
      (B) TYPE: amino acid
      (D) TOPOLOGY: linear
   (ii) MOLECULE TYPE: peptide
   (xi) SEQUENCE DESCRIPTION: SEQ ID NO: 1:
   Xaa Val Xaa Pro Xaa
(2) INFORMATION FOR SEQ ID NO: 2:
   (i) SEQUENCE CHARACTERISTICS:
      (A) LENGTH: 5 amino acids
      (B) TYPE: amino acid
      (D) TOPOLOGY: linear
   (ii) MOLECULE TYPE: peptide
   (xi) SEQUENCE DESCRIPTION: SEQ ID NO: 2:
   Xaa Ile Xaa Pro Xaa
(2) INFORMATION FOR SEQ ID NO: 3:
   (i) SEQUENCE CEARACTERISTICS
      (A) LENGTH: 5 amino acids
      (B) TYPE: amino acid
      (D) TOPOLOGY: linear
   (ii) MOLECULE TYPE: peptide
   (xi) SEQUENCE DESCRIPTION: SEQ ID NO: 3:
   Xaa Xaa Xaa Pro Xaa
(2) INFORMATION FOR SEQ ID NO: 4:
   (i) SEQUENCE CHARACTERISTICS:
      (A) LENGTH: 5 amino acids
      (B) TYPE: amino acid
      (D) TOPOLOGY: linear
   (ii) MOLECULE TYPE: peptide
   (xi) SEQUENCE DESCRIPTION: SEQ ID NO: 4:
   Xaa Val Xaa Xaa Xaa
(2) INFORMATION FOR SEQ ID NO: 5:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (xi) SEQUENCE DESCRIPTION: SEQ ID NO: 5:
   Xaa Val Xaa Pro Xaa Xaa